# EUROPEAN PATENT APPLICATION

(11) **EP 4 851 128 A1**
(43) Date of publication of application: **09.12.2026**
(21) Application number: 24921914.8
(22) Date of filing: 24.12.2024
(51) Int. Cl.: C07C 39/23, C07C 37/16, C07C 37/82, C07C 37/84, C07C 233/05

(54) **SOLVATE OF CANNABIDIOL AND METHOD FOR PURIFYING CANNABIDIOL**

(30) Priority: 29.01.2024 JP 2024010823
(71) Applicant: Shionogi Pharma Co., Ltd., Settsu-shi, Osaka 566-0022 (JP); Japan Tobacco Inc., Tokyo 105-6927 (JP)
(72) Inventor: SATO, Yusuke, Amagasaki-shi, Hyogo 660-0813 (JP); MORIYAMA, Mizuki, Amagasaki-shi, Hyogo 660-0813 (JP)
(74) Representative: Kraus & Lederer PartGmbB
(86) International application number: PCT/JP2024/045606
(87) International publication number: WO 2025/164161

(57) **Abstract**

A solvate of cannabidiol is provided.

A solvate of cannabidiol, wherein the solvate includes a solvent S1 including an aprotic polar solvent.

## Description

### TECHNICAL FIELD

The present invention relates to a solvate of cannabidiol, a method for purifying cannabidiol, and the like.

### BACKGROUND ART

Cannabidiol (CBD) is, for example, represented by the following formula, is one of the cannabinoids contained in hemp, and is used in various applications such as pharmaceuticals.

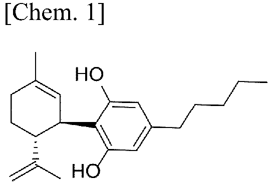

For example, Patent Literature 1 discloses a stable pharmaceutical preparation for oral administration comprising about 0.1% to about 40% cannabinoids and about 10% to about 95% lipids.

### CITATION LIST

### PATENT LITERATURE

Patent Literature 1: JP-A 2018-516281

### SUMMARY OF INVENTION

### TECHNICAL PROBLEM

An object of the present invention is to provide a solvate of cannabidiol and the like.

### SOLUTION TO PROBLEM

Cannabidiol (CBD) can be chemically (artificially) synthesized, for example, by reacting menthadienol with olivetol; however, such a reaction usually results in the formation of by-products (side products, reaction side products) in addition to CBD.

Examples of such by-products mainly include a positional isomer (hereinafter referred to as abn-CBD), an adduct (an adduct of menthadienol and olivetol at a molar ratio of 2:1, hereinafter referred to as 2:1 adduct), and tetrahydrocannabinol (THC) [in particular Δ9-THC (Δ⁹-THC) and Δ8-THC (Δ⁸-THC)] (for example, as represented by the following formulae). 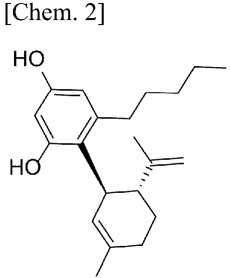 Positional isomer (abn-CBD) 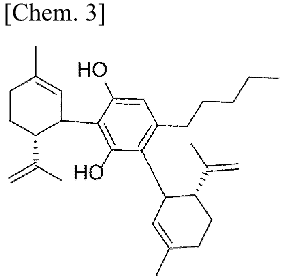 Adduct (2:1 adduct) 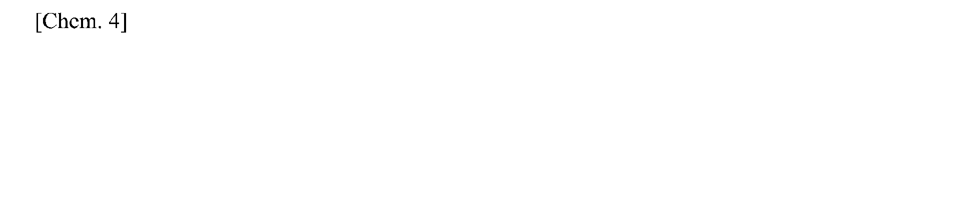 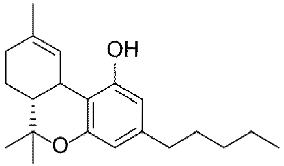 Δ9-THC 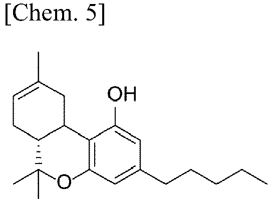 Δ8-THC

The generation of such by-products results in a reduction in CBD yield (obtained amount) as well as a reduction in efficiency of CBD recovery (purification).

Further, according to the studies conducted by the present inventors, generation of such by-products (for example, generation of relatively large amounts of by-products or generation of by-products that are difficult to separate from CBD), would cause difficulty in separation (purification) even by conventional purification techniques, or may make the separation (purification) process complicated even if separation (purification) is possible.

Insufficient separation results in the coexistence of by-products with CBD which may inhibit the functional expression of CBD, or may cause different pharmacological effects or side effects (for example, THCs are principal components of marijuana).

Therefore, it is preferable to suppress the formation of such by-products as much as possible at the reaction stage.

However, it is difficult to investigate reaction (production) conditions that suppress such by-products generation while producing CBD; in particular, suppressing the generation of one by-product may lead to the generation of another by-product. Accordingly, it has been extremely difficult to investigate conditions that suppress by-products as a whole (e.g., conditions that simultaneously suppress the formation of abn-CBD and the 2:1 adduct as well as the formation of THC).

Further, it is difficult to achieve suppression of by-product generation (suppression of by-product generation at a high level) while allowing olivetol to be reacted completely. Accordingly, there were cases where unreacted olivetol remained in the reaction mixture.

Under these circumstances, the present inventors have found that CBD can be produced while efficiently suppressing the generation of by-products by, for example, reacting menthadienol with olivetol under specific conditions (methods), and that CBD can be efficiently purified (other components can be separated from CBD) by, for example, subjecting a mixture comprising CBD and other components such as olivetol and abn-CBD to a specific treatment.

According to the method found by the present inventors, components (olivetol, by-products) other than CBD can be separated at a high level. However, even by such a method, complete separation of other components is difficult, and there is a case where CBD is produced with slight amounts (trace amounts) of other components.

Further studies by the present inventors revealed the causes specific to CBD production, such as limited number of solvents having poor solubility for CBD, structural similarity between CBD and by-products, and inhibition of CBD crystallization by highly polar components (such as olivetol). Therefore, unless other components are separated at an extremely high level, it may be difficult to obtain highly purified CBD by conventional methods such as crystallization, or it may be difficult to efficiently obtain CBD with sufficient yield or recovery efficiency.

Moreover, such problems may arise similarly even when CBD is not chemically (artificially) synthesized. For example, CBD derived from natural sources (e.g., CBD purified from hemp) is free from (or is unlikely to contain) components derived solely from chemical synthesis (e.g., starting material components such as olivetol), but may still contain other components {for example, cannabinoids other than CBD [e.g., cannabigerol (CBG), cannabinol (CBN), cannabichromene (CBC), cannabidivarin (CBDV), THCs (Δ9-THC etc.)], and the like}. In particular, many of these components cannot be readily separated from CBD (or are difficult to separate from CBD) by conventional methods such as crystallization, or even if separation is possible, it is extremely inefficient and impractical in many cases.

Under these circumstances, the present inventors have further investigated a possibility of purification of CBD from a mixture (crude CBD) containing CBD and other components (trace amounts of other components) from a completely different perspective, and have found that CBD is capable of forming a solvate with a specific solvent, and that formation or utilization of such a solvate facilitates efficient separation of other components. As a result of further investigation, the present invention has been completed.

That is, the present invention relates to the following inventions and others.
[1] A solvate of cannabidiol, wherein the solvate comprises a solvent S1 comprising an aprotic polar solvent.
[2] The solvate according to [1], wherein the solvent S1 comprises an amide-based solvent (amide).
[3] The solvate according to [1] or [2], wherein the solvent S1 comprises dimethylacetamide.
[4] The solvate according to any one of [1] to [3], which is a solvate having a molar ratio of 2 mol of dimethylacetamide relative to 1 mol of cannabidiol.
[5] The solvate according to any one of [1] to [4], which is in crystalline form (crystals).
[6] A method for producing a solvate of cannabidiol, the method comprising:
   forming a solvate from a mixture comprising cannabidiol and a solvent S1 (solvent S1 mixture), wherein the solvent S1 of the solvate comprises an aprotic polar solvent.
[7] The method according to [6], wherein the solvent S1 comprises an amide-based solvent (amide).
[8] The method according to [6] or [7], wherein the solvent S1 comprises dimethylacetamide, and the solvate is a solvate in which dimethylacetamide is solvated at a ratio of 2 mol relative to 1 mol of cannabidiol.
[9] The method according to any one of [6] to [8], wherein the mixture further comprises a solvent S2 different from the solvent S1.
[10] The method according to any one of [6] to [9], wherein the mixture further comprises an aliphatic hydrocarbon.
[11] The method according to any one of [6] to [10], wherein the mixture comprises dimethylacetamide and an aliphatic hydrocarbon, and precipitates (crystallizes) a solvate in which dimethylacetamide is solvated at a ratio of 2 mol relative to 1 mol of cannabidiol (to obtain a precipitate, solid, or crystal comprising the solvate; to form the solvate in the form of a precipitate, solid, or crystal comprising the solvate).
[12] The method according to any one of [6] to [11], wherein the mixture further comprises an additional cannabinoid (a cannabinoid other than cannabidiol).
[13] The method according to any one of [6] to [12], wherein the mixture comprises at least one additional cannabinoid selected from the group consisting of cannabigerol, cannabinol, cannabichromene, cannabidivarin, and tetrahydrocannabinol.
[14] The method according to any one of [6] to [13], wherein the mixture further comprises at least one cannabinoid selected from the group consisting of cannabigerol, cannabinol, Δ9-tetrahydrocannabinol, and Δ8-tetrahydrocannabinol, and
   the proportion of cannabigerol, cannabinol, Δ9-tetrahydrocannabinol, and Δ8-tetrahydrocannabinol relative to the total amount of cannabidiol, cannabigerol, cannabinol, Δ9-tetrahydrocannabinol, and Δ8-tetrahydrocannabinol is 0.1% or more on an HPLC area basis.
[15] The method according to any one of [6] to [14], wherein the mixture (or cannabidiol) is derived from hemp, in particular cannabis.
[16] The method according to any one of [6] to [15], wherein the mixture further contains at least one selected from the group consisting of olivetol, a positional isomer of cannabidiol (abn-CBD), an adduct of menthadienol and olivetol at a molar ratio of 2:1 (2:1 adduct), Δ9-tetrahydrocannabinol (Δ9-THC), and Δ8-tetrahydrocannabinol (Δ8-THC).
[17] The method according to any one of [6] to [16],
   wherein the mixture comprises cannabidiol (CBD) and an adduct of menthadienol and olivetol at a molar ratio of 2:1 (2:1 adduct),
   the proportion of cannabidiol (CBD) relative to the total amount of cannabidiol (CBD), olivetol, a positional isomer of cannabidiol (abn-CBD), the adduct (2:1 adduct), Δ9-tetrahydrocannabinol (Δ9-THC), and Δ8-tetrahydrocannabinol (Δ8-THC) is 60% or more on an HPLC area basis,
   the mixture is free from or contains olivetol at a proportion of 0.03 or less relative to cannabidiol (CBD) on an HPLC area basis,
   the mixture is free from or contains the positional isomer of cannabidiol (abn-CBD) at a proportion of 0.3 or less relative to cannabidiol (CBD) on an HPLC area basis, and
   the mixture contains the adduct (2:1 adduct) at a proportion of 0.0001 to 0.5 relative to cannabidiol (CBD) on an HPLC area basis.
[18] The method according to any one of [6] to [17], further comprising:
   subjecting a mixture comprising cannabidiol and at least one selected from the group consisting of olivetol and a positional isomer of cannabidiol (abn-CBD) to adsorption treatment to produce a purified product comprising cannabidiol, and
   forming a solvate from a mixture comprising thus-obtained purified product and the solvent S1 (solvent S1 mixture).
[19] The method according to any one of [6] to [18], further comprising:
   reacting menthadienol with olivetol in the presence of at least one catalyst (acid, acid catalyst) selected from the group consisting of a non-boron Lewis acid and a non-boron Bronsted acid (e.g., an organic acid, an inorganic acid) at a temperature of 40°C or more, with a water content in the reaction system at the start of the reaction of 5 mass% or less (e.g., 4 mass% or less, 3 mass% or less, 2 mass% or less, 1.5 mass% or less, 1 mass% or less, 0.5 mass% or less, 0.3 mass% or less, 0.25 mass% or less, 0.2 mass% or less, 0.18 mass% or less, 0.15 mass% or less, 0.12 mass% or less, 0.1 mass% or less, 0.08 mass% or less, or 0.05 mass% or less) to produce a reaction mixture comprising cannabidiol,
   subjecting thus-obtained reaction mixture to adsorption treatment to produce a purified product comprising cannabidiol, and
   forming a solvate from a mixture comprising thus-obtained purified product and the solvent S1 (solvent S1 mixture).
[20] A method for producing (regenerating) cannabidiol comprising:
   desolvating the solvate according to any one of [1] to [5].
[21] A method for producing (regenerating) cannabidiol comprising:
   mixing (mixing to desolvate) the solvate according to any one of [1] to [5] with water.
[22] A method for extracting (liquid-liquid phase-separating) cannabidiol (desolvated cannabidiol) comprising:
   mixing (mixing to desolvate) the solvate according to any one of [1] to [5], water, and a hydrophobic solvent capable of dissolving cannabidiol to extract (liquid-liquid phase-separate) cannabidiol (desolvated cannabidiol) into a hydrophobic solvent layer (a hydrophobic solvent-containing layer, a layer separated from water, or a liquid-liquid phase-separated layer).
[23] A method for producing (regenerating) cannabidiol, comprising:
   mixing the solvate according to any one of [1] to [5], water, and a hydrophobic solvent capable of dissolving cannabidiol (mixing to desolvate) to extract (liquid-liquid phase-separate) cannabidiol (desolvated cannabidiol) into a hydrophobic solvent layer (a hydrophobic solvent-containing layer, a layer separated from water, or a liquid-liquid phase-separated layer); and
   crystallizing (e.g., by crystallization) the cannabidiol (regenerated cannabidiol) obtained through the cannabidiol obtained through the extraction step.
[24] Cannabidiol obtained by the method according to any one of [20] to [23].
[25] The cannabidiol according to [24], wherein the proportion of cannabidiol relative to the total amount of cannabidiol, cannabigerol, cannabinol, Δ9-tetrahydrocannabinol, and Δ8-tetrahydrocannabinol is 97% or more on an HPLC area basis.
[26] The cannabidiol according to [24] or [25], wherein the proportion of cannabidiol (CBD) relative to the total amount of cannabidiol (CBD), olivetol, a positional isomer of cannabidiol (abn-CBD), an adduct of menthadienol and olivetol at a molar ratio of 2:1 (2:1 adduct), Δ9-tetrahydrocannabinol (Δ9-THC), and Δ8-tetrahydrocannabinol (Δ8-THC) is 97% or more on an HPLC area basis.
[27] The cannabidiol according to any one of [24] to [26], which is free from or contains a solvent S1 (e.g., dimethylacetamide) of the solvate at a proportion of 3 mass% or less.
[28] The cannabidiol according to any one of [24] to [27], containing a solvent S1 of the solvate at a proportion of 0.001 mass% or more.
[29] The cannabidiol according to any one of [24] to [28], containing a solvent S1 of the solvate at a proportion of 0.001 mass% or more and a hydrocarbon (e.g., an aliphatic hydrocarbon such as heptane) at a proportion of 0.001 mass% or more.

### ADVANTAGEOUS EFFECTS OF INVENTION

According to the present invention, a solvate of cannabidiol can be provided.

Such a solvate is useful, for example, for separating CBD (separating as a solvate) from a mixture comprising CBD and other components (trace amounts of other components) [crude CBD, or CBD containing other components (at trace amounts thereof) (a purified product or crude CBD)].

As described above, it is difficult to completely separate CBD from other components; however, such a solvate appears to be more readily separable from other components than CBD itself. Therefore, utilization of such a solvate (conversion of CBD contained in crude CBD into a solvate) achieves easier and more efficient separation from other components.

Moreover, while the solvate is stable in the form of the solvate, it can also be readily desolvated (and thus CBD can be regenerated).

Therefore, utilization of such a solvate also enables efficient (and even convenient) production to obtain CBD (in particular, high-purity CBD).

### DESCRIPTION OF EMBODIMENTS

### Solvate

The solvate of the present invention is a solvate of cannabidiol (CBD) (a CBD solvate, a solvate of CBD).

In the solvate of CBD, a solvent (which may also be referred to as a solvent of the solvate, a solvent forming the solvate, a solvent S1, or the like) may typically comprise an aprotic polar solvent.

In the solvate of CBD, specific examples of the solvent (solvent S1) include nitrogen-containing solvents {for example, amides [amide-based solvents, such as N-substituted chain-structured amides (e.g., N-substituted (e.g., N-alkyl) alkanamides such as N,N-dimethylformamide, N,N-dimethylacetamide; N,N-diphenylacetamide, etc.), cyclic amides (e.g., 2-pyrrolidone, 1,3-dimethyl-2-imidazolidinone, etc.), and the like], nitriles (e.g., acetonitrile), and the like]}, sulfur-containing solvents (e.g., sulfoxides such as dimethyl sulfoxide; sulfones such as dimethyl sulfone and sulfolane), and the like.

The solvate of CBD may comprise one or two or more solvents (e.g., aprotic polar solvents).

Further, in the solvate of CBD, the solvent (solvent S1) comprising an aprotic polar solvent may consist of the aprotic polar solvent, or may comprise a solvent other than the aprotic polar solvent. The solvent may particularly consist of an aprotic polar solvent.

In the solvate of CBD, preferred solvents include amides (amide-based solvents), in particular N-substituted alkanamides such as N,N-dimethylacetamide (dimethylacetamide), from the viewpoints of, for example, ease of precipitation from the mixture and ease of separation.

It should be noted that in the solvate of CBD, as long as a solvate is formed, the mole ratio (molar ratio) of CBD relative to the solvent (solvent S1) is not particularly limited.

The solvate of CBD may particularly be crystalline (in the form of a crystal).

As one embodiment of the solvate of CBD, there exemplified a solvate solvated with dimethylacetamide (which may be referred to as DMA).

In the solvate of CBD solvated with dimethylacetamide (which may be referred to as CBD·DMA, CBD·DMA solvate, CBD-DMA, or the like), the mole ratio of CBD relative to dimethylacetamide is not limited. The solvate may be, for example, a solvate comprising CBD and dimethylacetamide at a molar ratio of 2 mol of dimethylacetamide relative to 1 mol of CBD (which may be referred to as CBD-2DMA, CBD·2DMA, or the like); (the solvate may comprise at least CBD-2DMA).

Such CBD-2DMA may exhibit, for example, at least one of the following physical properties (characteristics).

### • Powder X-ray diffraction (2θ)

The CBD-2DMA may have at least the characteristic peaks shown in (A) below, or has at least the characteristic peaks shown in (B) or (C) below (the characteristic peaks including the peaks shown in (A) below).

### (A) (approximately 5 peaks)

8.3 ± 0.2°, 8.5 ± 0.2°, 10.6 ± 0.2°, 16.4 ± 0.2°, 17.0 ± 0.2°

### (B) (approximately 10 peaks)

8.3 ± 0.2°, 8.5 ± 0.2°, 10.6 ± 0.2°, 13.4 ± 0.2°, 16.4 ± 0.2°, 17.0 ± 0.2°, 18.9 ± 0.2°, 22.7 ± 0.2°, 24.6 ± 0.2°, 26.5 ± 0.2°

### (C) (approximately 20 peaks)

8.3 ± 0.2°, 8.5 ± 0.2°, 10.6 ± 0.2°, 11.8 ± 0.2°, 13.4 ± 0.2°, 15.4 ± 0.2°, 15.8 ± 0.2°, 16.4 ± 0.2°, 17.0 ± 0.2°, 18.9 ± 0.2°, 19.9 ± 0.2°, 21.4 ± 0.2°, 22.7 ± 0.2°, 22.9 ± 0.2°, 23.2 ± 0.2°, 23.7 ± 0.2°, 24.6 ± 0.2°, 26.5 ± 0.2°, 27.1 ± 0.2°, 27.7 ± 0.2°

- ¹H-NMR (for example, 400MHz, CDCl₃):δppm: 0.88 (t, J = 7.2Hz, 3H), 1.22-1.36 (m, 2H), 1.52-1.60 (m, 2H), 1.64-1.67 (m, 3H), 1.75-1.85 (m, 2H), 1.78-1.80 (m, 3H), 2.07-2.13 (m, 2H), 2.08 (s, 6H), 2.18-2.28 (m, 3H), 2.37-2.46 (m, 3H), 2.94 (s, 6H), 3.01 (s, 6H), 3.82-3.87 (m, 1H), 4.56 (s, 1H), 4.65-4.67 (m, 1H), 5.56-5.58 (m, 1H), 6.13-6.30 (m, 1H)
- Melting point: 52 to 55°C [determined by DSC (differential scanning calorimetry)]

It should be noted that CBD-2DMA generally has lower solubility than CBD in various solvents such as heptane. Accordingly, CBD-2DMA generally appears to crystallize more readily than CBD. Therefore, such a solvate is particularly effective for efficiently separating (purifying) CBD from a mixture comprising CBD and other components (e.g., by-products, olivetol).

Further, CBD-2DMA appears to release (desolvate) DMA relatively readily in water or the like. Accordingly, such a solvate is also effective in terms of forming (regenerating) CBD.

### Method for Producing Solvate of CBD

The solvate of CBD is not particularly limited and can be produced by contacting (mixing) CBD with a solvent of the solvate (a solvent forming the solvate, solvent S1).

Typically, the solvate can be formed from a mixture comprising CBD and a solvent S1 (solvent S1 mixture). In particular, the solvate can be precipitated (crystallized) from a mixture comprising CBD and the solvent S1 (solvent S1 mixture), in other words, a precipitate/solid/crystal comprising the solvate can be obtained, or the solvate can be formed in the form of a precipitate/solid/crystal comprising the solvate). Such a method facilitates efficient separation of CBD as a solvate.

As the CBD (CBD used in the production, CBD source) there may be used CBD (high-purity CBD), or a mixture comprising CBD and other components (trace amounts of other components).

In particular, when a mixture comprising CBD and other components is used (especially when a solvate is precipitated from a mixture comprising CBD and other components), CBD can be readily separated as a solvate from the other components.

The other components may vary depending on the origin of CBD [e.g., derived from natural sources (such as hemp, etc.), derived from chemical synthesis, etc.], and may include, for example, cannabinoids (cannabinoids other than CBD, additional cannabinoids), olivetol, menthadienol, abn-CBD, the 2:1 adducts, and the like.

Examples of additional cannabinoids may include cannabigerol (CBG), cannabinol (CBN), cannabichromene (CBC), cannabidivarin (CBDV), THCs (Δ8-THC, Δ9-THC, Δ10-THC, etc.), cannabidiolic acid (CBDA), cannabigerolic acid (CBGA), tetrahydrocannabinolic acid (THCA), tetrahydrocannabivarin (THCV), cannabicyclol (CBL), cannabinodiol (CBND), cannabielsoin (CBE), cannabitriol (CBT), and the like.

The other components may be present in the mixture singly or in combination of two or more.

Among these other components, in particular, additional cannabinoids (e.g., CBG, CBN, CBC, CBDV, THCs, and especially CBG, CBN, THCs), olivetol, abn-CBD, the 2:1 adduct, and the like are likely to be present in the mixture.

It should be noted that olivetol, menthadienol, abn-CBD, and the 2:1 adduct are often contained in CBD (mixture) derived from chemical synthesis, while other cannabinoids may be contained in the mixture regardless of whether they are derived from natural sources or from chemical synthesis.

The mixture comprising CBD and other components is not particularly limited, and the mixture may include a mixture from natural source [e.g., extracts of hemp (cannabis, etc.), purified products of such extracts (mixtures with enhanced proportion of CBD), and the like], a mixture derived from the reaction (chemical synthesis) (a mixture obtained by chemical synthesis), and the like.

Examples of mixtures from natural sources (other components in natural sources) include mixtures comprising other cannabinoids (a mixture comprising CBD and additional cannabinoids), and the like.

Examples of mixtures derived from chemical synthesis include a reaction mixture, a first purified product (crude CBD), and the like as described below. In particular, between the reaction mixture and the first purified product, it is preferable to use the first purified product (crude product, the first purified product having undergone the adsorption treatment described below), from the viewpoints of, for example, ease of precipitating the CBD solvate and ease of efficiently obtaining high-purity CBD.

The solvent S1 mixture is not particularly limited as long as it contains at least the solvent S1 as a solvent. From the viewpoints of, for example, ease of efficiently forming (precipitating) the CBD solvate, the solvent S1 mixture may comprise a solvate solvent (e.g., amides such as DMA) and another solvent (which may be referred to as a solvent S2 or the like) different from the solvate solvent, etc.); (the solvent S1 may be used in combination with the solvent S2).

It should be noted that the solvent S2 is not particularly limited as long as it does not form a solvate in combination with the solvent S1. The solvent S2 itself may be a solvent being capable of forming a solvate.

The solvent S2 may be a solvent different from the solvent S1, and is preferably a solvent that does not (substantially) dissolve the CBD solvate (a poor solvent for the CBD solvate).

The solvent S2 may be selected depending on the type of the solvent S1 and the solvate, and examples of the solvent S2 include hydrocarbons [e.g., aliphatic hydrocarbons (such as pentane, hexane, isohexane, heptane, 2-methylhexane, 2,2-dimethylpentane, octane, isooctane, nonane, decane, cyclopentane, cyclohexane, cycloheptane, and methylcyclohexane), aromatic hydrocarbons (such as benzene, toluene, xylene, and ethylbenzene)], halogenated solvents (e.g., halogenated hydrocarbons such as dichloromethane, chloroform, and carbon tetrachloride), ketones (e.g., acetone, methyl ethyl ketone, methyl isobutyl ketone, etc.), esters (e.g., aliphatic esters such as methyl acetate, ethyl acetate, and butyl acetate), carbonates (e.g., ethylene carbonate, propylene carbonate, etc.), ethers [e.g., chain-structured ethers (such as diethyl ether and methyl tert-butyl ether), cyclic ethers (such as tetrahydrofuran and dioxane) etc.], glycol ethers (e.g., ethylene glycol monomethyl ether, ethylene glycol monoethyl ether, propylene glycol monomethyl ether, diethylene glycol monoethyl ether, etc.), glycol ether esters (e.g., ethylene glycol monomethyl ether acetate etc.), nitrogen-containing solvents {e.g., amides [such as N-substituted chain-structured amides (e.g., N-alkyl-substituted alkanamides such as N,N-dimethylformamide), cyclic amides (e.g., 2-pyrrolidone etc.), etc.], etc.}, nitriles (e.g., acetonitrile etc.), sulfur-containing solvents (e.g., sulfoxides such as dimethyl sulfoxide; sulfones such as dimethyl sulfone and sulfolane), alcohols [e.g., alkanols (such as methanol, ethanol and isopropanol) etc.], polyols (e.g., alkanediols such as ethylene glycol and propylene glycol; glycerol etc.), etc.], water, and the like.

Typical examples of the solvent S2 include hydrocarbons [e.g., aliphatic hydrocarbons (such as pentane, hexane, isohexane, heptane, 2-methylhexane, 2,2-dimethylpentane, octane, isooctane, nonane, decane, cyclopentane, cyclohexane, cycloheptane, and methylcyclohexane), aromatic hydrocarbons (such as benzene, toluene, xylene, and ethylbenzene) etc.], ketones (e.g., acetone, methyl ethyl ketone, methyl isobutyl ketone, etc.), ethers [e.g., chain-structured ethers (such as diethyl ether and methyl tert-butyl ether), cyclic ethers (such as tetrahydrofuran and dioxane), etc.], and the like.

Among them, the solvent S2 may particularly comprise at least an aliphatic hydrocarbon {e.g., aliphatic hydrocarbons [e.g., aliphatic hydrocarbons (e.g., chain-structured or cyclic aliphatic hydrocarbons, saturated or unsaturated hydrocarbons, chain-structured or cyclic saturated aliphatic hydrocarbons, etc.) having 5 or more carbon atoms (e.g., 5 to 18 carbon atoms, 6 to 12 carbon atoms, 6 to 10 carbon atoms, etc.) etc.] such as pentane, hexane, isohexane, heptane, 2-methylhexane, 2,2-dimethylpentane, octane, isooctane, nonane, decane, cyclopentane, cyclohexane, cycloheptane, and methylcyclohexane, and the like}.

The solvent S2 may be used singly or in combination of two or more.

When the solvent S2 comprises an aliphatic hydrocarbon, the proportion of the aliphatic hydrocarbon (e.g., heptane, etc.) in the total amount of the solvent S2 may be selected from a range of, for example, about 1 vol% or more (e.g., 3 vol% or more), preferably 5 vol% or more (e.g., 10 vol% or more), more preferably 15 vol% or more (e.g., 20 vol% or more), and may be 25 vol% or more [e.g., 30 vol% or more, 35 vol% or more, 40 vol% or more, 50 vol% or more, greater than 50 vol%, 60 vol% or more, 70 vol% or more, 80 vol% or more, 85 vol% or more, 90 vol% or more, 95 vol% or more, (substantially) 100 vol%, or the solvent S2 consisting of (consisting essentially of) an aliphatic hydrocarbon].

The aliphatic hydrocarbon may be used in combination with an additional solvent (a solvent other than aliphatic hydrocarbons, a solvent such as an ether). In such a case, the proportion of the additional solvent in the total amount of the solvent S2 may be selected from a range of, for example, 99 vol% or less (e.g., 97 vol% or less, 95 vol% or less), and may be 90 vol% or less (e.g., 80 vol% or less), preferably 70 vol% or less (e.g., 60 vol% or less), more preferably 50 vol% or less (e.g., 40 vol% or less), and may be 1 vol% or more (e.g., 3 vol% or more, 5 vol% or more, or 10 vol% or more).

The proportion of the solvent S1 in the total amount of the solvent is not particularly limited, and may be, for example, 0.1 vol% or more (e.g., 0.5 vol% or more), preferably 1 vol% or more (e.g., 2 vol% or more), more preferably 3 vol% or more (e.g., 5 vol% or more).

When the solvent comprises the solvent S2, the proportion of the solvent S2 in the total amount of the solvent is not particularly limited, and may be selected from a range of, for example, about 1 vol% or more (e.g., 3 vol% or more), preferably about 5 vol% or more (e.g., 10 vol% or more), more preferably about 15 vol% or more (e.g., 20 vol% or more), and may be 25 vol% or more (e.g., 30 vol% or more, 35 vol% or more, 40 vol% or more, 50 vol% or more, greater than 50 vol%, 60 vol% or more, 70 vol% or more, 80 vol% or more, 85 vol% or more, or 90 vol% or more).

It should be noted that the solvent (e.g., solvent S2) may or may not contain a protic solvent (e.g., water, alcohols, etc.). From the viewpoint of, for example, ease of efficiently forming (precipitating) the CBD solvate, the solvent preferably is free from or contains a protic solvent in only a small amount [e.g., the amount of a protic solvent being 10 vol% or less (e.g., 5 vol% or less, 3 vol% or less, 1 vol% or less, 0.5 vol% or less, 0.1 vol% or less) in the total solvent]. In particular, the solvent may be free from or contain water only in a small amount [e.g., the amount of water being 10 vol% or less (e.g., 5 vol% or less, 3 vol% or less, 1 vol% or less, 0.5 vol% or less, or 0.1 vol% or less) in the total solvent].

In the mixture (the solvent S1 mixture), the proportion (concentration) of components other than the solvent (solid components or non-solvent components) relative to the total amount of the solvent and the non-solvent components (components other than the solvent) is not particularly limited, and may be, for example, about 0.1 mass% or more (e.g., 0.5 mass% or more, 1 mass% or more, 3 mass% or more, or 5 mass% or more), and may be 99 mass% or less (e.g., 95 mass% or less, 90 mass% or less, 80 mass% or less, 70 mass% or less, 60 mass% or less, 50 mass% or less, 45 mass% or less, 40 mass% or less, 35 mass% or less, 30 mass% or less, 25 mass% or less, 20 mass% or less, 15 mass% or less, 12 mass% or less, or 10 mass% or less).

In the mixture (the solvent S1 mixture), the proportion of the solvent per 1 mol of CBD by volume may be about 0.01 or more (e.g., 0.05 or more, 0.1 or more, 0.3 or more, or 0.5 or more), and may be 1000 or less (e.g., 800 or less, 500 or less, 300 or less, 200 or less, 150 or less, 120 or less, 100 or less, 80 or less, 70 or less, 60 or less, 50 or less, or 40 or less).

In the mixture, the proportion of the solvent S1 relative to CBD may be, for example, about 1 molar equivalent or more, greater than 1 molar equivalent (e.g., 1.05 molar equivalents), preferably 1.1 molar equivalents or more (e.g., 1.2 molar equivalents or more), more preferably 1.5 molar equivalents or more (e.g., 1.8 molar equivalents or more), and may be 2 molar equivalents or more (e.g., 2.5 molar equivalents or more, or 3 molar equivalents or more).

It should be noted that "one molar equivalent" refers to X mol of a solvent relative to 1 mol of CBD in a solvate, when the solvate is formed with X mol of the solvent relative to 1 mol of CBD (e.g., 2 mol of DMA per 1 mol of CBD in the case of CBD-2DMA).

The solvate of CBD can be formed (produced) in a mixture (solvent S1 mixture) by reacting (contacting) CBD with the solvent S1. The formation of such a solvate may generally be accompanied by crystal formation (crystallization).

In such a reaction (solvation), the method of mixing raw (starting) materials (components) to be used is not particularly limited. All components may be mixed together, mixed stepwise, partially mixed, or a plurality of components may be pre-mixed, or any combination thereof may be employed.

In particular, from the viewpoints of, for example, ease of efficiently forming the solvate of CBD, the solvent S1 may be mixed (e.g., added dropwise) into a system (mixture) comprising CBD (e.g., a reaction mixture or first purified product) [solvation may be carried out by mixing (e.g., adding dropwise) the solvent S1].

The mixing may be carried out at once or stepwise, and may preferably be carried out stepwise. Such mixing (stepwise mixing) may be carried out intermittently, continuously (successively), or a combination thereof.

Furthermore, in such mixing (stepwise mixing), the time required for mixing (mixing time, total time, time until completion of mixing) depends on factors such as the amount of the other component(s), and may be selected from a range of, for example, about 30 seconds or more (e.g., 40 seconds or more), and may be 1 minute or more (e.g., 2 minutes or more, 3 minutes or more, 5 minutes or more, 10 minutes or more, or 30 minutes or more).

The reaction (crystallization) may be carried out in the absence or presence of seed crystals (seeds to initiate crystallization). The use of seed crystals often facilitates crystallization under mild conditions (e.g., crystallization readily occurs without the need to reduce the temperature, etc.). The seed crystals may be, for example, a solvate of CBD. Such seed crystals may be those prepared in advance, for example, prepared (by the method of the present invention) from CBD with high purity, or those prepared (by the method of the present invention) from a mixture comprising CBD and other components (e.g., a reaction mixture or first purified product, etc.).

The reaction (crystallization) may be carried out at room temperature (or ambient temperature), under heating, or under cooling.

Examples of the reaction (crystallization) temperature include, for example, -100°C or more (e.g., -80°C or more, -60°C or more, -40°C or more, -20°C or more, -10°C or more, 0°C or more, 10°C or more, 20°C or more, 30°C or more, 35°C or more, or 40°C or more) and 150°C or less (e.g., 140°C or less, 130°C or less, 120°C or less, 110°C or less, 100°C or less, 90°C or less, 80°C or less, 70°C or less, 60°C or less, or 50°C or less).

In particular, from the viewpoints of, for example, ease of efficiently forming the solvate and ease of precipitating the solvate, the reaction (crystallization) temperature may be set to a relatively low or not excessively high temperature (e.g., 40°C or less, 35°C or less, 30°C or less, 25°C or less, 20°C or less, 15°C or less, 10°C or less, or 5°C or less).

The reaction (crystallization) time may be appropriately selected depending on factors such as the degree of progress of solvation (crystallization) and various reaction (crystallization) conditions, and is not particularly limited, and may be, for example, 30 seconds or more (e.g., 1 minute or more, 2 minutes or more, 5 minutes or more, 10 minutes or more, 15 minutes or more, 20 minutes or more, 25 minutes or more, 30 minutes or more, 40 minutes or more, 60 minutes or more, 1.5 hours or more, or 2 hours or more), and may be 48 hours or less (e.g., 36 hours or less, 24 hours or less, 18 hours or less, 12 hours or less, 6 hours or less, 5 hours or less, 4 hours or less, or 3 hours or less).

The reaction (crystallization) may be carried out with stirring or under stationary conditions.

The reaction (crystallization) may also be carried out under an inert atmosphere (e.g., nitrogen or argon) and the like.

The vessel used in the reaction (crystallization) is not particularly limited, and conventional vessels may be used.

Through the above reaction (crystallization), a solvate of CBD can be formed (produced).

The separation (recovery) of the solvate of CBD from the reaction (crystallization) mixture can be carried out by conventional methods. For example, the crystal-formed (crystallized, precipitated) solvate of CBD (crystals, crystallization product, solid components or solid fraction, solid) may be separated (recovered) by filtration, decantation, or the like.

It should be noted that the separated solvate of CBD (crystals, crystallization product, solid components or solid fraction, solid) may be washed (subjected to a washing treatment) as needed.

The washing solvent is not particularly limited, and one or more of the solvents exemplified above (e.g., solvent S2) may be used. Preferred embodiments of solvents are also the same as those described above For example, a solvent comprising a hydrocarbon (in particular, aliphatic hydrocarbon), preferably a solvent comprising at least an aliphatic hydrocarbon may be used.

The amount of washing solvent may be appropriately selected depending on the amount of the solvate of CBD (crystals, crystallization product, solid components or solid fraction, solid), and may be, for example, 0.1 parts by mass or more (e.g., 0.3 parts by mass or more, 0.5 parts by mass or more, or 1 part by mass or more), may be 100 parts by mass or less (e.g., 50 parts by mass or less, 10 parts by mass or less, 5 parts by mass or less) relative to 1 part by mass of the solvate of CBD (crystal or crystalline product).

It should be noted that CBD can be substantially recovered as the solvate of CBD in the solid (crystals, crystallization product, solid components or solid fraction, second purified product (e.g., purified product obtained via the first purified product), solvate of CBD). For example, when A1 denotes the mass of CBD in the mixture (solvent S1 mixture) and A2 denotes the mass of CBD in the second purified product (the mass attributable to CBD in the solvate), the value of "A2/A1" can be selected from a range of, for example, about 0.7 or more, and may be about 0.8 or more (e.g., 0.85 or more, 0.88 or more, 0.9 or more, 0.92 or more, 0.93 or more, 0.94 or more, 0.95 or more, 0.96 or more, 0.97 or more, 0.98 or more, 0.99 or more, or substantially 1).

Further, the solid (crystals, crystallization product, solid components or solid fraction, second purified product, solvate of CBD) may or may not contain other components (e.g., components derived from the reaction (chemical synthesis) such as olivetol and by-products; other cannabinoids, etc.). By undergoing the purification (crystallization, solvate formation) as described above, even when such components are present, they are often present only in trace amounts (i.e., the solid is highly purified).

For example, in the solid (crystals, crystallization product, solid components or solid fraction, second purified product, solvate of CBD), the proportion [for example, the proportion based on an HPLC (high-performance liquid chromatography) area (or peak area, for example, an area measured at a detection wavelength of 210 nm; the same applies hereinafter to the HPLC area), or on an HPLC area basis] of CBD (or a solvate of CBD) relative to the total amount of CBD (or the solvate of CBD), olivetol, abn-CBD, the 2:1 adduct, Δ9-THC, and Δ8-THC may be selected from a range of, for example, 80% or more (e.g., 85% or more), and may be 88% or more (e.g., 89% or more), preferably 90% or more (e.g., 91% or more), more preferably 92% or more (e.g., 93% or more), and in particular 94% or more (e.g., 95% or more, 96% or more, 96.5% or more, 97% or more, 97.5% or more, 98% or more, 98.5% or more, 99% or more, 99.2% or more, 99.5% or more, or 99.9% or more).

In the solid (crystals, crystallization product, solid components or solid fraction, second purified product, solvate of CBD, in particular those derived from CBD derived from natural sources), the proportion (e.g., the proportion on an HPLC area (peak area) basis) of CBD (or a solvate of CBD) relative to the total amount of CBD (or the solvate of CBD) and other cannabinoids [or CBG, CBN, CBC, CBDV, and THCs (e.g., Δ9-THC, Δ8-THC)] [or CBG, CBN, and THCs (e.g., Δ9-THC, Δ8-THC)] may be selected from a range of, for example, 80% or more (e.g., 85% or more), and may be 88% or more (e.g., 89% or more), preferably 90% or more (e.g., 91% or more), more preferably 92% or more (e.g., 93% or more), and particularly 94% or more (e.g., 95% or more, 96% or more, 96.5% or more, 97% or more, 97.5% or more, 98% or more, 98.5% or more, 99% or more, 99.2% or more, 99.5% or more, or 99.9% or more).

In the mixture (e.g., a mixture comprising CBD derived from natural sources, a mixture comprising other cannabinoids, a mixture prior to second purification, or a solvent S1 mixture), when M1 denotes the proportion (e.g., the proportion on an HPLC area (peak area) basis) of CBD relative to the total amount of CBD, olivetol, abn-CBD, the 2:1 adduct, Δ9-THC, and Δ8-THC, and M2 denotes the proportion (e.g., the proportion on an HPLC area (peak area) basis) of CBD (or a solvate of CBD) relative to the total amount of CBD (or the solvate of CBD), olivetol, abn-CBD, the 2:1 adduct, Δ9-THC, and Δ8-THC in the second purified product, the value of "M2 - M1" may be greater than 0% and may be selected from a range of about 1% or more (e.g., 2% or more), and may be 3% or more (e.g., 4% or more), preferably 5% or more (e.g., 8% or more), more preferably 10% or more (e.g., 12% or more), and particularly 15% or more (e.g., 16% or more, 17% or more, 18% or more, 19% or more, or 20% or more).

In the mixture (e.g., a mixture comprising CBD derived from natural sources, a mixture comprising other cannabinoids, a mixture prior to second purification, or a solvent S1 mixture), when M1 denotes the proportion (e.g., the proportion on an HPLC area (peak area) basis) of CBD relative to the total amount of CBD (or a solvate of CBD) and other cannabinoids [or CBG, CBN, CBC, CBDV, and THCs (e.g., Δ9-THC, Δ8-THC)] [or CBG, CBN, and THCs (e.g., Δ9-THC, Δ8-THC)], and M2 denotes the proportion (e.g., the proportion on an HPLC area (peak area) basis) of CBD (ora solvate of CBD) relative to the total amount of CBD (or the solvate of CBD) and other cannabinoids [or CBG, CBN, CBC, CBDV, and THCs (e.g., Δ9-THC, Δ8-THC)] [or CBG, CBN, and THCs (e.g., Δ9-THC, Δ8-THC)] in the solid (crystals, crystallization product, solid components or solid fraction, second purified product, solvate of CBD, in particular those derived from natural sources), the value of "M2 - M1" may be greater than 0% and may be selected from a range of about 1% or more (e.g., 2% or more), and may be 3% or more (e.g., 4% or more), preferably 5% or more (e.g., 8% or more), more preferably 10% or more (e.g., 12% or more), and particularly 15% or more (e.g., 16% or more, 17% or more, 18% or more, 19% or more, or 20% or more).

The solid (crystals, crystallization product, solid components or solid fraction, second purified product, solvate of CBD, in particular the second purified product) may or may not contain olivetol. Even when olivetol is present, olivetol is often present only in a small amount. For example, in the solid (crystals, crystallization product, solid components or solid fraction, second purified product, solvate of CBD, in particular the second purified product), the proportion (e.g., the proportion on an HPLC area basis) of olivetol relative to the total amount of CBD (or a solvate of CBD), olivetol, abn-CBD, the 2:1 adduct, Δ9-THC, and Δ8-THC may be, for example, 10% or less (e.g., 8% or less, 7% or less, 6% or less, 5% or less, 4% or less, 3% or less, 2% or less, 1.5% or less, 1.2% or less, 1% or less, 0.9% or less, 0.8% or less, 0.7% or less, 0.6% or less, 0.5% or less, 0.4% or less, 0.3% or less, 0.2% or less, or 0.1% or less).

In the mixture (e.g., a mixture comprising CBD derived from natural sources, a mixture comprising other cannabinoids, a mixture prior to second purification, or a solvent S1 mixture), when M1 denotes the proportion (e.g., the proportion on an HPLC area (peak area) basis) of olivetol relative to the total amount of CBD, olivetol, abn-CBD, the 2:1 adduct, Δ9-THC, and Δ8-THC, and M2 denotes the proportion (e.g., the proportion on an HPLC area (peak area) basis) of olivetol relative to the total amount of CBD (or a solvate of CBD), olivetol, abn-CBD, the 2:1 adduct, Δ9-THC, and Δ8-THC in the second purified product, the value of "M1 - M2" may be greater than 0% and may be selected from a range of about 0.01% or more (e.g., 0.02% or more), and may be 0.03% or more (e.g., 0.05% or more), preferably 0.07% or more (e.g., 0.08% or more), more preferably 0.1% or more (e.g., 0.12% or more or 0.15% or more), and may be 0.2% or more (e.g., 0.5% or more, 1% or more, 1.5% or more, 2% or more, 2.5% or more, 3% or more, or 3.5% or more).

The solid (crystals, crystallization product, solid components or solid fraction, second purified product, solvate of CBD, in particular the second purified product) may or may not contain abn-CBD. Even when the abn-CBD is present, abn-CBD is often present only in a small amount. For example, in the solid (crystals, crystallization product, solid components or solid fraction, second purified product, solvate of CBD, in particular the second purified product), the proportion (e.g., the proportion on an HPLC area basis) of abn-CBD relative to the total amount of CBD (or a solvate of CBD), olivetol, abn-CBD, the 2:1 adduct, Δ9-THC, and Δ8-THC may be, for example, 10% or less (e.g., 8% or less, 7% or less, 6% or less, 5% or less, 4% or less, 3% or less, 2% or less, 1.5% or less, 1% or less, 0.9% or less, 0.8% or less, 0.7% or less, 0.6% or less, 0.5% or less, 0.4% or less, or 0.3% or less).

In the mixture (e.g., a mixture comprising CBD derived from natural sources, a mixture comprising other cannabinoids, a mixture prior to second purification, or a solvent S1 mixture), when M1 denotes the proportion (e.g., the proportion on an HPLC area (peak area) basis) of abn-CBD relative to the total amount of CBD, olivetol, abn-CBD, the 2:1 adduct, Δ9-THC, and Δ8-THC, and M2 denotes the proportion (e.g., the proportion on an HPLC area (peak area) basis), of abn-CBD relative to the total amount of CBD (or a solvate of CBD), olivetol, abn-CBD, the 2:1 adduct, Δ9-THC, and Δ8-THC in the second purified product the value of "M1 - M2" may be greater than 0% and may be selected from a range of about 0.1% or more (e.g., 0.5% or more), and may be 1% or more (e.g., 1.2% or more), preferably 1.5% or more (e.g., 1.8% or more), more preferably 2% or more (e.g., 2.2% or more), and particularly 2.5% or more (e.g., 2.8% or more, 3% or more, 3.2% or more, 3.5% or more, 3.8% or more, or 4% or more).

The solid (crystals, crystallization product, solid components or solid fraction, second purified product, solvate of CBD, in particular the second purified product) may or may not contain the 2:1 adduct. Even when the 2:1 adduct is present, the 2:1 adduct is often present only in a small amount. For example, in the solid (crystals, crystallization product, solid components or solid fraction, second purified product, solvate of CBD, in particular the second purified product), the proportion (e.g., the proportion on an HPLC area basis) of the 2:1 adduct relative to the total amount of CBD (or a solvate of CBD), olivetol, abn-CBD, the 2:1 adduct, Δ9-THC, and Δ8-THC may be, for example, 3% or less (e.g., 2.5% or less, 2% or less, 1.5% or less, 1.2% or less, 1% or less, 0.9% or less, 0.8% or less, 0.7% or less, 0.6% or less, 0.5% or less, 0.4% or less, 0.3% or less, 0.2% or less, 0.1% or less, 0.05% or less, 0.01% or less, or 0.0001% or less).

The solid (crystals, crystallization product, solid components or solid fraction, second purified product, solvate of CBD, in particular those derived from natural sources) may or may not contain other cannabinoids. Even when other cannabinoids are present, such cannabinoids are often present only in small amounts. For example, in the solid (crystals, crystallization product, solid components or solid fraction, second purified product, solvate of CBD, in particular those derived from natural sources), and in the solid (crystal, crystallized product, solid components or solid fraction, second purified product, solvate of CBD, in particular those derived from CBD derived from natural sources), the proportion (e.g., the proportion on an HPLC area (peak area) basis) of other cannabinoids [or CBG, CBN, CBC, CBDV, and THCs (e.g., Δ9-THC, Δ8-THC)] [or CBG, CBN, and THCs (e.g., Δ9-THC, Δ8-THC)] relative to the total amount of CBD (or a solvate of CBD) and other cannabinoids [or CBG, CBN, CBC, CBDV, and THCs (e.g., Δ9-THC, Δ8-THC)] [or CBG, CBN, and THCs (e.g., Δ9-THC, Δ8-THC)] may be, for example, 20% or less (e.g., 18% or less, 16% or less, 15% or less, 12% or less, 10% or less, 9% or less, 8% or less, 7% or less, 6% or less, 5% or less, 4% or less, 3% or less, 2% or less, 1.5% or less, 1.2% or less, 1% or less, 0.9% or less, 0.8% or less, 0.7% or less, 0.6% or less, 0.5% or less, 0.4% or less, 0.3% or less, 0.2% or less, or 0.1% or less).

The solid (crystals, crystallization product, solid components or solid fraction, second purified product, solvate of CBD, in particular the second purified product) may or may not contain Δ9-THC. Even when Δ9-THC is present, the Δ9-THC is often present only in a small amount. For example, in the solid (crystals, crystallization product, solid components or solid fraction, second purified product, solvate of CBD, in particular the second purified product), the proportion (e.g., the proportion on an HPLC area basis) of Δ9-THC relative to the total amount of CBD (or a solvate of CBD), olivetol, abn-CBD, the 2:1 adduct, Δ9-THC, and Δ8-THC may be, for example, 1% or less (e.g., 0.9% or less, 0.8% or less, 0.7% or less, 0.6% or less, 0.5% or less, 0.4% or less, 0.3% or less, 0.2% or less, 0.1% or less, 0.05% or less, 0.01% or less, or 0.0001% or less).

The solid (crystal, crystallization product, solid components or solid fraction, second purified product, solvate of CBD, in particular the second purified product) may or may not contain Δ8-THC. Even if Δ8-THC is present, the Δ8-THC is often present only in a small amount. For example, in the solid (crystal, crystallization product, solid components or solid fraction, second purified product, solvate of CBD, in particular the second purified product), the proportion (e.g., the proportion on an HPLC area basis) of Δ8-THC relative to the total amount of CBD (or a solvate of CBD), olivetol, abn-CBD, the 2:1 adduct, Δ9-THC, and Δ8-THC may be, for example, 1% or less (e.g., 0.9% or less, 0.8% or less, 0.7% or less, 0.6% or less, 0.5% or less, 0.4% or less, 0.3% or less, 0.2% or less, 0.1% or less, 0.05% or less, 0.01% or less, or 0.0001% or less).

The solid (crystal, crystallization product, solid components or solid fraction, second purified product, solvate of CBD, in particular the second purified product) may or may not contain Δ9-THC and Δ8-THC. Even if Δ9-THC and Δ8-THC are present, they are often present only in small amounts. For example, in the solid (crystal, crystallization product, solid components or solid fraction, second purified product, solvate of CBD, in particular the second purified product), the proportion (e.g., the proportion on an HPLC area basis) of Δ9-THC and Δ8-THC relative to the total amount of CBD (or a solvate of CBD), olivetol, abn-CBD, the 2:1 adduct, Δ9-THC, and Δ8-THC may be, for example, 1% or less (e.g., 0.9% or less, 0.8% or less, 0.7% or less, 0.6% or less, 0.5% or less, 0.4% or less, 0.3% or less, 0.2% or less, 0.1% or less, 0.05% or less, 0.01% or less, or 0.0001% or less).

When the solid (crystals, crystallization product, solid components or solid fraction, second purified product, solvate of CBD, in particular the second purified product) comprises olivetol, in the solid (crystal, crystallization product, solid components or solid fraction, second purified product, or solvate of CBD, in particular the second purified product), the proportion (e.g., the proportion on an HPLC area basis) of olivetol relative to CBD (or a solvate of CBD) (i.e., the proportion of olivetol/the proportion of CBD (or the solvate of CBD)) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, or 0.001 or less).

When the solid (crystal, crystallization product, solid components or solid fraction, second purified product, solvate of CBD, in particular the second purified product) comprises abn-CBD, in the solid (crystal, crystallization product, solid components or solid fraction, second purified product, or solvate of CBD, in particular the second purified product), the proportion (e.g., the proportion on an HPLC area basis) of abn-CBD relative to CBD (or a solvate of CBD) (i.e., the proportion of abn-CBD/the proportion of CBD (or the solvate of CBD)) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less).

When the solid (crystal, crystallization product, solid components or solid fraction, second purified product, solvate of CBD, in particular the second purified product) comprises the 2:1 adduct, in the solid (crystal, crystallization product, solid components or solid fraction, second purified product, or solvate of CBD, in particular the second purified product), the proportion (e.g., the proportion on an HPLC area basis) of the 2:1 adduct relative to CBD (or a solvate of CBD) (i.e., the proportion of the 2:1 adduct/the proportion of CBD (or the solvate of CBD)) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, 0.0005 or less, or 0.0001 or less).

When the solid (crystal, crystallization product, solid components or solid fraction, second purified product, solvate of CBD, in particular the second purified product) comprises Δ9-THC, in the solid (crystal, crystallization product, solid components or solid fraction, second purified product, or solvate of CBD, in particular the second purified product), the proportion (e.g., the proportion on an HPLC area basis) of Δ9-THC relative to CBD (or a solvate of CBD) (i.e., the proportion of Δ9-THC/the proportion of CBD (or the solvate of CBD)) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, 0.0005 or less, or 0.0001 or less).

When the solid (crystal, crystallization product, solid components or solid fraction, second purified product, solvate of CBD, in particular the second purified product) comprises Δ8-THC, in the solid (crystal, crystallization product, solid components or solid fraction, second purified product, or solvate of CBD, in particular the second purified product), the proportion (e.g., the proportion on an HPLC area basis) of Δ8-THC relative to CBD (or a solvate of CBD) (i.e., the proportion of Δ8-THC/the proportion of CBD (or the solvate of CBD)) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, 0.0005 or less, or 0.0001 or less).

When the solid (crystal, crystallization product, solid components or solid fraction, second purified product, solvate of CBD, in particular the second purified product) comprises Δ9-THC and Δ8-THC, in the solid (crystal, crystallization product, solid components or solid fraction, second purified product, or solvate of CBD, in particular the second purified product), the proportion (e.g., the proportion on an HPLC area basis) of Δ9-THC and Δ8-THC relative to CBD (or a solvate of CBD) (i.e., the proportion of Δ9-THC and Δ8-THC/the proportion of CBD (or the solvate of CBD)) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, 0.0005 or less, or 0.0001 or less).

In the solid (crystals, crystallization product, solid components or solid fraction, second purified product, solvate of CBD, in particular the second purified product), the proportion (e.g., the proportion on an HPLC area basis) of olivetol, abn-CBD, the 2:1 adduct, Δ9-THC, and Δ8-THC relative to CBD (or a solvate of CBD) [i.e., (the sum of the proportions of olivetol, abn-CBD, the 2:1 adduct, Δ9-THC, and Δ8-THC)/the proportion of CBD (or the solvate of CBD)]may be selected from a range of, for example, about 0.5 or less (e.g., 0.3 or less), and may be 0.1 or less (e.g., 0.08 or less), preferably 0.05 or less (e.g., 0.03 or less), more preferably 0.02 or less (e.g., 0.015 or less), and particularly 0.01 or less (e.g., 0.008 or less, 0.006 or less, or 0.004 or less).

### Mixture comprising CBD and other (additional) components

As described above, in the production of a solvate of CBD, a mixture comprising CBD and other components (trace amounts of other components) (such as a mixture derived from natural sources and a mixture derived from the reaction) may be used as a source of the CBD (CBD to be used in the production, CBD source). Hereinafter, such mixtures [primarily mixtures derived from the reaction (chemical synthesis) (mixtures obtained through chemical synthesis)] will be described in detail.

Examples of other components include unreacted starting materials (e.g., olivetol, menthadienol), by-products (abn-CBD, the 2:1 adduct, Δ9-THC, Δ8-THC, etc.), other components used in the reaction (e.g., a catalyst), and the like. The other components may also be additional cannabinoids.

The other components may typically comprise at least one selected from the group consisting of olivetol and by-products (abn-CBD, the 2:1 adduct, Δ9-THC, Δ8-THC, etc.). Such other components are often contained in reaction-derived mixtures.

Typically, the mixture may comprise, as other components, an additional cannabinoid [for example, one selected from CBG, CBN, CBC, CBDV, and THCs (e.g., Δ9-THC, Δ8-THC), and in particular, one selected from CBG, CBN, and THCs (e.g., Δ9-THC, Δ8-THC)]. Although additional cannabinoids are often contained in mixtures derived from natural sources, such additional cannabinoids (e.g., THCs) are also often contained in mixtures derived from the reaction described above.

One embodiment of such mixtures (thereof) include a mixture containing an additional cannabinoid. Such a mixture may be either a mixture derived from natural sources or a mixture derived from the reaction, and may particularly be a mixture derived from natural sources.

The mixture comprising an additional cannabinoid may be, for example, one obtained by separating (purifying) CBD from a raw material derived from natural sources (e.g., hemp), or may be a commercially available product.

In such a mixture, the proportion (e.g., the proportion on an HPLC area (peak area) basis) of CBD relative to the total amount of CBD and additional cannabinoids [or CBG, CBN, CBC, CBDV, and THCs (e.g., Δ9-THC, Δ8-THC)] [or CBG, CBN, and THCs (e.g., Δ9-THC, Δ8-THC)] may be selected from a range of, for example, 40% or more (e.g., 45% or more), and may be 50% or more (e.g., 52% or more), preferably 55% or more (e.g., 58% or more), more preferably 60% or more (e.g., 62% or more), and particularly 65% or more (e.g., 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, or 95% or more), and may be 99.99% or less (e.g., 99.9% or less, 99.8% or less, 99.5% or less, 99% or less, 98% or less, 97% or less, 95% or less, 92% or less, 90% or less, or 88% or less).

In the mixture (a mixture comprising additional cannabinoids), the proportion (e.g., the proportion on an HPLC area (peak area) basis) of additional cannabinoids [or CBG, CBN, CBC, CBDV, and THCs (e.g., Δ9-THC, Δ8-THC)] [or CBG, CBN, and THCs (e.g., Δ9-THC, Δ8-THC)] relative to the total amount of CBD and the additional cannabinoids [or CBG, CBN, CBC, CBDV, and THCs (e.g., Δ9-THC, Δ8-THC)] [or CBG, CBN, and THCs (e.g., Δ9-THC, Δ8-THC)] may be selected from a range of, for example, 0.01% or more (e.g., 0.03% or more), and may be 0.05% or more (e.g., 0.1% or more), preferably 0.2% or more (e.g., 0.3% or more), more preferably 0.5% or more (e.g., 0.7% or more or 0.8% or more), and particularly 1% or more (e.g., 1.2% or more, 1.3% or more, 1.4% or more, 1.5% or more, 1.6% or more, 1.7% or more, 1.8% or more, 1.9% or more, 2% or more, 2.5% or more, 3% or more, 3.5% or more, 4% or more, or 5% or more), and may be 20% or less (e.g., 18% or less, 15% or less, 12% or less, 10% or less, 9% or less, 8% or less, 7% or less, 6% or less, 5% or less, 4.5% or less, 4% or less, 3.5% or less, 3% or less, 2.8% or less, 2.5% or less, 2.2% or less, 2% or less, 1.5% or less, or 1% or less).

Further, examples of such mixtures (one embodiment of mixtures, mixtures derived from the reaction) include a reaction mixture, a first purified product (crude CBD), and the like. In particular, between the reaction mixture and the first purified product (e.g., a purified product obtained by subjecting the reaction mixture to adsorption treatment), the first purified product (a crude product, a first purified product having undergone the adsorption treatment described below) may preferably be used from the viewpoints of, for example, ease of precipitating the CBD solvate and ease of efficiently obtaining high-purity CBD.

Hereinafter, such a mixture (in particular, a mixture derived from the reaction) comprising CBD and other (additional) components (trace amounts of other (additional) components), particularly mixtures derived from the reaction, will be described in detail.

### Reaction mixture

The reaction mixture (a reaction mixture comprising CBD, CBD) is not particularly limited, and is generally obtained by reacting menthadienol with olivetol. CBD is produced (formed) by the reaction.

The amount (use ratio) of menthadienol to olivetol is not particularly limited and may be an equimolar ratio, or either may be used in excess. For example, relative to 1 mol of menthadienol, the amount of olivetol may be selected from a range of about 0.3 mol or more (e.g., 0.5 mol or more), and may be 0.6 mol or more (e.g., 0.7 mol or more), preferably 0.8 mol or more (e.g., 0.85 mol or more), more preferably 0.9 mol or more (e.g., 0.95 mol or more, 0.97 mol or more, or 0.99 mol or more), and may be 1 mol or more (e.g., more than 1 mol, 1.01 mol or more, 1.02 mol or more, 1.03 mol or more, 1.04 mol or more, or 1.05 mol or more).

The olivetol amount (upper limit) relative to 1 mol of menthadienol is not particularly limited, and may be selected from a range of about 5 mol or less (e.g., 4 mol or less), preferably about 3 mol or less (e.g., 2 mol or less), more preferably about 1.5 mol or less (e.g., 1.3 mol or less, 1.25 mol or less, 1.2 mol or less, 1.15 mol or less, 1.12 mol or less, or 1.1 mol or less), and may be 1 mol or less (e.g., 0.99 mol or less, 0.98 mol or less, 0.97 mol or less, 0.95 mol or less, 0.93 mol or less, or 0.9 mol or less).

It should be noted that the amount of olivetol relative to 1 mol of menthadienol may be expressed as a range combining the upper limit and the lower limit (e.g., 0.3 mol to 1 mol, or greater than 1 mol and not greater than 1.5 mol); the same applies hereinafter.

In the present invention, the reaction may be carried out in the presence of (using) a specific catalyst [at least one selected from the group consisting of a non-boron-based Lewis acid and a Bronsted acid (a protonic acid)].

Examples of the non-boron-based Lewis acids include non-boron-based compounds such as oxides (e.g., metal oxides), halides (e.g., fluorides, chlorides, bromides, iodides, etc.) (e.g., metal halides), alkoxides (e.g., metal alkoxides), triflates (e.g., metal triflates), and other complexes (e.g., acetylacetonate complexes, acetoacetate ester complexes) and the like.

Examples of the metals (non-boron-based metals, metals other than boron) include main-group metals (elements) and transition metals (transition elements), such as Group 1 to Group 17 metals (elements) of the periodic table, including, for example, metals (elements) of Group 3 to 12 of the periodic table (e.g., scandium, yttrium, lanthanum, titanium, zirconium, hafnium, vanadium, niobium, tantalum, chromium, molybdenum, tungsten, manganese, iron, ruthenium, cobalt, rhodium, iridium, nickel, palladium, platinum, copper, silver, gold, zinc, cadmium, etc.), metals (elements) of Group 13 to 16 metals of the periodic table (e.g., aluminum, gallium, indium, thallium, tin, lead, arsenic, antimony, bismuth, etc.) and the like.

Specific examples of the non-boron-based Lewis acids include oxides (e.g., metal oxides such as aluminum oxide), halides [e.g., metal halides such as zinc halides (e.g., zinc chloride, zinc bromide, zinc iodide, etc.)], alkoxides [e.g., metal alkoxides such as titanium alkoxides (titanium tetramethoxide, titanium tetraethoxide, etc.), zirconium alkoxide, etc.], triflates (e.g., metal triflates such as scandium triflate, silver triflate, and zinc triflate), acetylacetonate complexes (e.g., metal acetylacetonates such as aluminum acetylacetonate), acetoacetate ester complexes (e.g., metal acetoacetate ester complexes such as aluminum ethyl acetoacetate diisopropylate), and the like.

Examples of Bronsted acids include organic acids, inorganic acids, and the like.

Examples of organic acids include carboxylic acids {e.g., aliphatic carboxylic acids [e.g., monocarboxylic acids (e.g., alkanoic monocarboxylic acids) such as formic acid, acetic acid, propionic acid, lactic acid, and trifluoroacetic acid; and polycarboxylic acids (e.g., alkanoic polycarboxylic acids) such as oxalic acid, malonic acid, succinic acid, glutaric acid, tartaric acid, malic acid, and citric acid], and aromatic carboxylic acids (e.g., benzoic acid, phthalic acid, terephthalic acid, etc.) etc.}, sulfonic acids (e.g., alkanesulfonic acids such as methanesulfonic acid and trifluoromethanesulfonic acid; arenesulfonic acids such as benzenesulfonic acid and p-toluenesulfonic acid), and the like.

Examples of inorganic acids include hydrogen halides (e.g., hydrogen chloride, hydrogen bromide, hydrogen iodide), sulfuric acid, nitric acid, phosphoric acid, and the like.

The catalyst (acid catalyst) may be used singly or in combination of two or more.

Typical catalysts include non-boron-based Lewis acids [e.g., metal oxides, metal halides, metal triflates], carboxylic acids, sulfonic acids, inorganic acids, and the like.

Among these catalysts, non-boron-based Lewis acids may preferably be used from the viewpoints of, for example, suppressing the formation of by-products and achieving good reactivity (and further, readily and efficiently achieving suppression of by-product formation and good reactivity in combination with other conditions). Among them, in particular, metal halides (e.g., zinc halides such as zinc chloride, zinc bromide and zinc iodide), may preferably be used.

Therefore, the catalyst may comprise at least a non-boron-based Lewis acid [e.g., a metal halide (e.g., a zinc halide such as zinc chloride, zinc bromide, and zinc iodide, and in particular zinc iodide)].

As described above, the reaction is generally carried out in the presence of a specific catalyst [at least one selected from the group consisting of a non-boron-based Lewis acid and a Bronsted acid (protonic acid)].

In such a case, the specific catalyst may be used in combination with a boron-based Lewis acid [e.g., a boron trifluoride complex (e.g., boron trifluoride diethyl ether complex, boron trifluoride monoalkylamine complex, boron trifluoride hydrate, etc.)]. On the other hand, it may be not necessary for the specific catalyst to be used in such a combination [used in combination, or substantially used (used in combination)] with a boron-based Lewis acid from the viewpoints of, for example, suppressing the formation of by-products and achieving good reactivity (and further, readily and efficiently achieving suppression of by-product formation and good reactivity in combination with other conditions).

The amount (amount used) of the catalyst (specific catalyst) may be selected depending on factors such as the type of catalyst. For example, relative to 1 mol of menthadienol or olivetol (e.g., menthadienol), the amount of the catalyst may be selected from a range of about 0.0001 mol (or molar equivalent, the same applies hereinafter) or more. From the viewpoints of, for example, achieving suppression of by-product formation and good reactivity (and readily and efficiently achieving suppression of by-product formation and good reactivity in combination with other conditions), the amount of the catalyst may be about 0.001 mol or more (e.g., 0.003 mol or more), preferably about 0.005 mol or more (e.g., 0.007 mol or more), more preferably about 0.01 mol or more (e.g., 0.03 mol or more), and may be about 0.05 mol or more (e.g., 0.07 mol or more, 0.08 mol or more, or 0.1 mol or more).

The amount (upper limit) of the catalyst (specific catalyst), relative to 1 mol of menthadienol or olivetol (e.g., menthadienol), may be selected from a range of, for example, about 20 mol or less (e.g., 15 mol or less, 10 mol or less, 8 mol or less). From the viewpoints of, for example, suppressing the formation of by-products and achieving good reactivity (and further, readily and efficiently achieving suppression of by-product formation and good reactivity in combination with other conditions), the amount (upper limit) may be about 5 mol or less (e.g., 3 mol or less), preferably about 2 mol or less (e.g., 1.2 mol or less), more preferably about 1 mol or less (e.g., less than 1 mol or 0.8 mol or less), and may be 0.5 mol or less (e.g., 0.4 mol or less, 0.3 mol or less, 0.2 mol or less, 0.15 mol or less, or 0.1 mol or less).

The reaction may be carried out in the presence of a solvent (in a solvent).

Examples of the solvent include hydrocarbons [e.g., aliphatic hydrocarbons (such as hexane, heptane, cyclohexane, and methylcyclohexane), aromatic hydrocarbons (such as benzene, toluene, xylene, and ethylbenzene)], halogenated solvents (e.g., halogenated hydrocarbons such as dichloromethane, chloroform, and carbon tetrachloride), ketones (e.g., acetone, methyl ethyl ketone, methyl isobutyl ketone, etc.), esters (e.g., aliphatic esters such as methyl acetate, ethyl acetate, and butyl acetate), carbonates (e.g., ethylene carbonate, propylene carbonate, etc.), ethers [e.g., chain-structured ethers (such as diethyl ether and methyl tert-butyl ether), cyclic ethers (e.g., tetrahydrofuran, dioxane, etc.) etc.], glycol ethers (e.g., ethylene glycol monomethyl ether, ethylene glycol monoethyl ether, propylene glycol monomethyl ether, diethylene glycol monoethyl ether, etc.), glycol ether esters (e.g., ethylene glycol monomethyl ether acetate, etc.), nitrogen-containing solvents {e.g., amides [e.g., N-substituted chain-structured amides (e.g., N-alkyl-substituted alkanamides such as N,N-dimethylformamide and N,N-dimethylacetamide; N,N-diphenylacetamide; etc.), cyclic amides (e.g., 2-pyrrolidone, 1,3-dimethyl-2-imidazolidinon, etc.), etc.], nitriles (e.g., acetonitrile etc.), etc.}, sulfur-containing solvents (e.g., sulfoxides such as dimethyl sulfoxide; sulfones such as dimethyl sulfone and sulfolane), alcohols [e.g., alkanols (such as methanol, ethanol, and isopropanol) etc.], polyols (e.g., alkanediols such as ethylene glycol and propylene glycol; glycerol etc.), etc.], water, and the like.

It should be noted that the Bronsted acids described above [e.g., organic acids (e.g., acetic acid etc.), etc.] may also be used as a solvent.

The solvent may be, for example, any solvent, including an aprotic solvent, a protic solvent (e.g., water, alcohols, and organic acids), a polar solvent, a nonpolar solvent, and the like. As the solvent, an aprotic solvent may preferably be used.

The solvent may be used singly or in combination of two or more.

Among these solvents, a solvent such as aprotic solvents may preferably be used from the viewpoints of, for example, suppression of by-product formation and good reactivity (and further, readily and efficiently achieving suppression of by-product formation and good reactivity in combination with other conditions). In particular, hydrocarbons (in particular, aromatic hydrocarbons such as toluene) may preferably be used.

Therefore, the solvent may comprise at least an aprotic solvent and/or a hydrocarbon (in particular, an aromatic hydrocarbon such as toluene).

The solvent may or may not contain a protic solvent (e.g., water, an alcohol, an organic acid, etc.). From the viewpoints of, for example, achieving suppression of by-product formation and good reactivity (and further readily and efficiently achieving suppression of by-product formation and good reactivity in combination with other conditions), it is preferable that the solvent is free from a protic solvent. Alternatively, if present, the protic solvent is preferably contained only in a small amount [e.g., relative to the amount of the solvent, 10 mass% or less (e.g., 5 mass% or less, 3 mass% or less, 1 mass% or less, 0.5 mass% or less, 0.1 mass% or less), etc.].

In particular, it is preferable that the solvent (reaction system) is free from or contains water only in a small amount, from the viewpoints of, for example, achieving suppression of by-product formation and good reactivity (and further, readily and efficiently achieving suppression of by-product formation and good reactivity in combination with other conditions).

When the solvent (reaction system) contains water, relative to the reaction system (e.g., the total amount of menthadienol, olivetol, catalyst, and solvent), the proportion of water may be selected from a range of about 10 mass% or less (e.g., 8 mass% or less), and may be 5 mass% or less (e.g., 4 mass% or less, 3.5 mass% or less), preferably 3 mass% or less (e.g., 2.5 mass% or less), more preferably 2 mass% or less (e.g., 1.5 mass% or less), particularly 1.2 mass% or less (e.g., 1 mass% or less, 0.9 mass% or less, 0.8 mass% or less, 0.7 mass% or less, 0.6 mass% or less, 0.5 mass% or less, 0.4 mass% or less, 0.3 mass% or less, 0.28 mass% or less), and particularly preferably 0.25 mass% or less (e.g., 0.22 mass% or less, 0.2 mass% or less, 0.18 mass% or less, 0.15 mass% or less, 0.12 mass% or less, 0.1 mass% or less, 0.08 mass% or less, 0.07 mass% or less, 0.06 mass% or less, 0.05 mass% or less, 0.04 mass% or less, 0.03 mass% or less, or 0.01 mass% or less).

When the solvent (reaction system) contains water, the proportion of water relative to the solvent may be selected from a range of about 10 mass% or less and may be 5 mass% or less (e.g., 3 mass% or less), preferably 2 mass% or less (e.g., 1.5 mass% or less), more preferably 1 mass% or less (e.g., 0.5 mass% or less, 0.1 mass% or less).

When the solvent (reaction system) contains water, the proportion of water relative to 100 parts by mass of menthadienol or olivetol (e.g., menthadienol) may be selected from a range of about 20 parts by mass or less (e.g., 15 parts by mass or less), and may be 10 parts by mass or less (e.g., 8 parts by mass or less), preferably 5 parts by mass or less (e.g., 3 parts by mass or less), more preferably 2 parts by mass or less (e.g., 1.5 parts by mass or less, 1 part by mass or less, 0.5 parts by mass or less, 0.3 parts by mass or less, or 0.1 parts by mass or less).

It should be noted that water is generated (formed as a by-product) by the reaction of menthadienol with olivetol. Therefore, the above-mentioned proportion of water may be the proportion of water before the reaction (or at the start of the reaction, or in the early stage of the reaction, or when the water generated as a by-product is removed).

Further, during the reaction, the proportion of water may be maintained (kept) at the relatively low proportion (range) as described above. For example, during the reaction, the proportion of water in the reaction system (e.g., the total amount of menthadienol, olivetol, catalyst, and solvent) or in the solvent may be maintained (kept) at 10 mass% or less (e.g., 5 mass% or less, 3 mass% or less, 2 mass% or less, 1 mass% or less, 0.8 mass% or less, 0.5 mass% or less, 0.4 mass% or less, 0.3 mass% or less, 0.2 mass% or less, 0.1 mass% or less).

It should be noted that a protic solvent (e.g., water) may be contained in advance in raw materials (components) used in the reaction [e.g., olivetol, a catalyst (e.g., a Bronsted acid), an aprotic solvent, etc.].

Accordingly, when the raw materials contain water in advance and it is necessary to reduce the amount of a protic solvent (e.g., water), the protic solvent may be separated or removed from the raw materials or the reaction system by conventional methods (e.g., distillation, etc.) (the proportion of water as described above may be adjusted by such separation or removal).

Further, as described above, although water is generated as a by-product in the reaction, the reaction may be carried out while separating and removing the generated water by an appropriate method.

When a solvent is used, the proportion (concentration) of components (solid components, e.g., menthadienol, olivetol, and catalyst) other than the solvent relative to the total amount of the solvent and the components other than the solvent may be, for example, about 0.1 mass% or more (e.g., 0.5 mass% or more, 1 mass% or more, 3 mass% or more, or 5 mass% or more), and may be 99 mass% or less (e.g., 95 mass% or less, 90 mass% or less, 80 mass% or less, 70 mass% or less, 60 mass% or less, 50 mass% or less, 45 mass% or less, 40 mass% or less, 35 mass% or less, 30 mass% or less, 25 mass% or less, 20 mass% or less, 15 mass% or less, 12 mass% or less, or 10 mass% or less).

When a solvent is used, the amount of the solvent relative to 1 part of olivetol or menthadienol (e.g., menthadienol) may be, by volume, about 0.01 or more (e.g., 0.05 or more, 0.1 or more, 0.3 or more, or 0.5 or more), and may be 1000 or less (e.g., 800 or less, 500 or less, 300 or less, 200 or less, 150 or less, 120 or less, 100 or less, 80 or less, 70 or less, 60 or less, 50 or less, or 40 or less).

The method of mixing the starting materials (components) (olivetol, menthadienol, catalyst, solvent, etc.) used in the reaction is not particularly limited. All components may be mixed together, mixed stepwise, mixed partially, or a plurality of components may be premixed, or any combination thereof may be employed.

In particular, into a system (mixture) containing one of menthadienol and olivetol (e.g., olivetol) and a catalyst (and optionally a solvent), the other of menthadienol and olivetol (e.g., menthadienol) may be mixed (e.g., added dropwise) [menthadienol and olivetol may be mixed (e.g., added dropwise) to react menthadienol with olivetol], from the viewpoints of, for example, achieving suppression of by-product formation and good reactivity (and further, readily and efficiently achieving suppression of by-product formation and good reactivity in combination with other conditions).

In particular, from the viewpoint of allowing the reaction to proceed efficiently (allowing the catalyst to function efficiently), a mixture comprising olivetol and a catalyst may be mixed with menthadienol (e.g., menthadienol may be added dropwise into a system (mixture) comprising olivetol and a catalyst).

The mixing may be carried out at once or stepwise, and may preferably be carried out stepwise.

Such mixing (stepwise mixing) may be carried out intermittently, continuously (successively), or a combination thereof.

Furthermore, in such mixing (stepwise mixing), the time required for mixing (mixing time, total time, time until completion of mixing) depends on factors such as the amount of the additional component(s), and may be selected from a range of, for example, about 30 seconds or more (e.g., 40 seconds or more), and may be 1 minute or more (e.g., 2 minutes or more), preferably 3 minutes or more (e.g., 4 minutes or more), more preferably 5 minutes or more (e.g., 7 minutes or more), and may be 8 minutes or more (e.g., 10 minutes or more, 12 minutes or more, 15 minutes or more, 18 minutes or more, 20 minutes or more, 25 minutes or more, or 30 minutes or more).

The reaction may be carried out at room temperature (or ambient temperature), under heating, or under cooling.

Examples of specific reaction temperatures include -100°C or more (e.g., -80°C or more, -60°C or more, -40°C or more, -20°C or more, 0°C or more, 10°C or more, 20°C or more, 30°C or more, 35°C or more, 40°C or more, 45°C or more, 50°C or more, 55°C or more, 60°C or more, 65°C or more, 70°C or more, 75°C or more, 80°C or more, 85°C or more, 90°C or more, 95°C or more, 100°C or more, 105°C or more, or 110°C or more), and 300°C or less (e.g., 250°C or less, 200°C or less, 180°C or less, 160°C or less, 150°C or less, 140°C or less, 130°C or less, 120°C or less, 110°C or less, 100°C or less, 95°C or less, 90°C or less, 85°C or less, or 80°C or less).

In particular, from the viewpoints of, for example, achieving suppression of by-product formation and good reactivity (and further, readily and efficiently achieving suppression of by-product formation and good reactivity in combination with other conditions), the reaction temperature may be set to a relatively high temperature (e.g., 30°C or more, 35°C or more, 40°C or more, 45°C or more, 50°C or more, 55°C or more, 60°C or more, 65°C or more, 70°C or more, 75°C or more, 80°C or more, 85°C or more, 90°C or more, 95°C or more, 100°C or more, 105°C or more, or 110°C or more).

The reaction time may be appropriately selected depending on factors such as the degree of the reaction progress (conversion, conversion rate), the type of catalyst, and various reaction conditions, is not particularly limited, and may be, for example, 1 second or more (e.g., 3 seconds or more, 5 seconds or more, 10 seconds or more, 20 seconds or more, 30 seconds or more, 40 seconds or more, 50 seconds or more, 1 minute or more, 1.5 minutes or more, 2 minutes or more, 2.5 minutes or more, 3 minutes or more, 3.5 minutes or more, 4 minutes or more, 4.5 minutes or more, 5 minutes or more, 8 minutes or more, 10 minutes or more, 15 minutes or more, 20 minutes or more, 25 minutes or more, or 30 minutes or more), and may be 24 hours or less (e.g., 18 hours or less, 12 hours or less, 10 hours or less, 8 hours or less, 6 hours or less, 5 hours or less, 4 hours or less, 3 hours or less, 2 hours or less, 1.5 hours or less, 1.2 hours or less, 1 hour or less, 50 minutes or less, 40 minutes or less, or 30 minutes or less).

It should be noted that the reaction is regarded as starting when menthadienol and olivetol (and further the catalyst) coexist in a state in which they can react. The reaction is regarded as ending when the reaction no longer proceeds (menthadienol or olivetol has completely reacted or has been completely consumed) or when an operation (treatment) for slowing (quenching) the reaction progress is performed.

For example, as described above, when the stepwise mixing is carried out, the reaction is regarded as starting when mixing is initiated. When the reaction is carried out under heating, the reaction is regarded as ending when heating is stopped or cooling is initiated; and the time from the start of the reaction to the end of the reaction is regarded as the reaction time.

It should be noted that the reaction may be carried out under stirring.

The reaction may also be carried out under an inert atmosphere (e.g., under nitrogen or argon) and the like.

The reaction may be carried out in batch mode or in continuous mode.

The reaction vessel used in the reaction is not particularly limited, and conventional vessels may be used.

Through the above reaction, cannabidiol is produced (manufactured). Cannabidiol is generally obtained as a mixture (a reaction mixture, a reaction solution) comprising cannabidiol. The present invention also encompasses such a mixture (a reaction mixture).

It should be noted that the mixture (a reaction mixture) may be concentrated (solvent-removed), filtered, or the like.

In the reaction mixture [after reaction (completion of the reaction); the reaction system after reaction (completion of the reaction)], the conversion (conversion rate) of menthadienol or olivetol [when one component (e.g., olivetol) is used in excess, generally the other component (e.g., menthadienol)] may be selected, depending on factors such as the timing of reaction completion, from a range of, for example, 20 mol% or more (e.g., 30 mol% or more, 40 mol% or more), and may be 50 mol% or more (e.g., 60 mol% or more), preferably 70 mol% or more (e.g., 80 mol% or more), more preferably 90 mol% or more (e.g., 95 mol% or more), and may be 97 mol% or more (e.g., 98 mol% or more, 99 mol% or more, or 100 mol%).

In the reaction mixture, the formation yield (yield) of cannabidiol (CBD) may be selected from a range of, for example, 10 mol% or more (e.g., 15 mol% or more), and may be 20 mol% or more (e.g., 25 mol% or more), preferably 30 mol% or more (e.g., 35 mol% or more), more preferably 40 mol% or more (e.g., 45 mol% or more), and particularly 50 mol% or more (e.g., 52 mol% or more, 55 mol% or more, 58 mol% or more, or 60 mol% or more).

The formation yield (upper limit thereof) of CBD may be selected from a range of, for example, about 99 mol% or less (e.g., 97 mol% or less, 95 mol% or less, 90 mol% or less), and is generally 85 mol% or less (e.g., 80 mol% or less, 75 mol% or less, 70 mol% or less, or 65 mol% or less).

It should be noted that the formation yield of CBD refers to the proportion (mol%) of menthadienol or olivetol that has reacted to form cannabidiol (i.e., has been converted into cannabidiol through reaction), relative to the amount of the menthadienol or olivetol used as a raw material [when one of the components (e.g., olivetol) is used in excess, the other component (e.g., menthadienol)].

The mixture (in particular, a reaction mixture) comprises CBD and other (additional) components, as described above.

In the mixture (a reaction mixture, etc.), the proportion (e.g., the proportion on an HPLC area (peak area) basis) of CBD relative to the total amount of CBD, olivetol, abn-CBD, the 2:1 adduct, Δ9-THC, and Δ8-THC may be selected from a range of, for example, 10% or more (e.g., 15% or more), and may be 20% or more (e.g., 25% or more), preferably 30% or more (e.g., 35% or more), more preferably 40% or more (e.g., 45% or more), particularly 50% or more (e.g., 52% or more, 55% or more, 58% or more, or 60% or more), and may be 99% or less (e.g., 97% or less, 95% or less, 90% or less, 85% or less, 80% or less, 75% or less, 70% or less, or 65% or less).

When the mixture (a reaction mixture, etc.) comprises olivetol, the proportion (e.g., the proportion on an HPLC area basis) of olivetol relative to the total amount of CBD, olivetol, abn-CBD, the 2:1 adduct, Δ9-THC, and Δ8-THC in the mixture may be selected from a range of, for example, 0.1% or more (e.g., 0.5% or more), and may be 1% or more (e.g., 2% or more), preferably 3% or more (e.g., 4% or more), more preferably 5% or more (e.g., 6% or more), particularly 7% or more (e.g., 8% or more, 9% or more, 10% or more, 11% or more, or 12% or more), and may be 50% or less (e.g., 45% or less, 40% or less, 35% or less, 30% or less, 25% or less, 22% or less, 20% or less, 18% or less, or 15% or less).

When the mixture (a reaction mixture, etc.) comprises abn-CBD, the proportion (e.g., the proportion on an HPLC area basis) of abn-CBD relative to the total amount of CBD, olivetol, abn-CBD, the 2:1 adduct, Δ9-THC, and Δ8-THC in the mixture may be selected from a range of, for example, 0.1% or more (e.g., 0.5% or more), and may be 1% or more (e.g., 2% or more), preferably 3% or more (e.g., 4% or more), more preferably 5% or more (e.g., 6% or more), particularly 7% or more (e.g., 8% or more, 9% or more, 10% or more, 11% or more, or 12% or more), and may be 70% or less (e.g., 65% or less, 60% or less, 55% or less, 50% or less, 45% or less, 40% or less, 35% or less, 30% or less, 25% or less, 22% or less, 20% or less, 18% or less, or 15% or less).

When the mixture (a reaction mixture, etc.) comprises olivetol and abn-CBD, the proportion (e.g., the proportion on an HPLC area basis) of olivetol and abn-CBD relative to the total amount of CBD, olivetol, abn-CBD, the 2:1 adduct, Δ9-THC, and Δ8-THC in the mixture may be selected from a range of, for example, 0.2% or more (e.g., 1% or more), and may be 2% or more (e.g., 4% or more), preferably 5% or more (e.g., 8% or more), more preferably 10% or more (e.g., 12% or more), particularly 15% or more (e.g., 16% or more, 18% or more, 20% or more, 22% or more, 24% or more, or 25% or more), and may be 80% or less (e.g., 70% or less, 65% or less, 60% or less, 55% or less, 50% or less, 45% or less, 40% or less, 35% or less, or 30% or less).

When the mixture (a reaction mixture, etc.) comprises the 2:1 adduct, the proportion (e.g., the proportion on an HPLC area basis) of the 2:1 adduct relative to the total amount of CBD, olivetol, abn-CBD, the 2:1 adduct, Δ9-THC, and Δ8-THC in the mixture may be selected from a range of, for example, 0.01% or more (e.g., 0.05% or more), and may be 0.1% or more (e.g., 0.3% or more), preferably 0.5% or more (e.g., 0.7% or more), more preferably 1% or more (e.g., 1.2% or more), and particularly 1.5% or more (e.g., 1.8% or more, 2% or more, 2.2% or more, 2.5% or more, 2.8% or more, 3% or more, 3.2% or more, 3.5% or more, 3.8% or more, 4% or more, 4.2% or more, 4.5% or more, 4.8% or more, 5% or more, 5.2% or more, or 5.5% or more), and may be 50% or less (e.g., 45% or less, 40% or less, 35% or less, 30% or less, 25% or less, 22% or less, 20% or less, 18% or less, 15% or less, 12% or less, 10% or less, 9% or less, 8% or less, 7% or less, 6% or less, or 5.5% or less).

When the mixture (a reaction mixture, etc.) comprises Δ9-THC, the proportion (e.g., the proportion on an HPLC area basis) of Δ9-THC relative to the total amount of CBD, olivetol, abn-CBD, the 2:1 adduct, Δ9-THC, and Δ8-THC in the mixture may be selected from a range of, for example, 0.01% or more (e.g., 0.03% or more), and may be 0.05% or more (e.g., 0.1% or more), preferably 0.2% or more (e.g., 0.3% or more), more preferably 0.5% or more (e.g., 0.7% or more), and particularly 0.8% or more (e.g., 1% or more, 1.2% or more, 1.3% or more, 1.4% or more, 1.5% or more, 1.6% or more, 1.7% or more, 1.8% or more, 1.9% or more, or 2% or more), and may be 20% or less (e.g., 18% or less, 15% or less, 12% or less, 10% or less, 9% or less, 8% or less, 7% or less, 6% or less, 5% or less, 4.5% or less, 4% or less, 3.5% or less, 3% or less, 2.8% or less, 2.5% or less, or 2.2% or less).

When the mixture (a reaction mixture, etc.) comprises Δ8-THC, the proportion (e.g., the proportion on an HPLC area basis) of Δ8-THC relative to the total amount of CBD, olivetol, abn-CBD, the 2:1 adduct, Δ9-THC, and Δ8-THC in the mixture may be selected from a range of, for example, 0.001% or more (e.g., 0.005% or more), and may be 0.01% or more (e.g., 0.03% or more), preferably 0.05% or more (e.g., 0.07% or more), more preferably 0.1% or more (e.g., 0.12% or more), particularly 0.15% or more (e.g., 0.18% or more, 0.2% or more, 0.22% or more, or 0.25% or more), and may be 10% or less (e.g., 5% or less, 3% or less, 2% or less, 1% or less, 0.9% or less, 0.8% or less, 0.7% or less, 0.6% or less, 0.55% or less, 0.5% or less, 0.45% or less, 0.4% or less, 0.35% or less, or 0.3% or less).

When the mixture (a reaction mixture, etc.) comprises Δ9-THC and Δ8-THC, the proportion (e.g., the proportion on an HPLC area basis) of Δ9-THC and Δ8-THC relative to the total amount of CBD, olivetol, abn-CBD, the 2:1 adduct, Δ9-THC, and Δ8-THC in the mixture may be selected from a range of, for example, 0.01% or more (e.g., 0.03% or more), and may be 0.05% or more (e.g., 0.1% or more), preferably 0.2% or more (e.g., 0.3% or more), more preferably 0.5% or more (e.g., 0.7% or more), particularly 0.8% or more (e.g., 1% or more, 1.2% or more, 1.3% or more, 1.4% or more, 1.5% or more, 1.6% or more, 1.7% or more, 1.8% or more, 1.9% or more, 2% or more, 2.1% or more, or 2.2% or more), and may be 20% or less (e.g., 18% or less, 15% or less, 12% or less, 10% or less, 9% or less, 8% or less, 7% or less, 6% or less, 5% or less, 4.5% or less, 4% or less, 3.5% or less, 3.2% or less, 3% or less, 2.8% or less, or 2.5% or less).

When the mixture (a reaction mixture, etc.) comprises olivetol, the proportion (e.g., the proportion on an HPLC area basis) of olivetol relative to CBD (i.e., the proportion of olivetol/the proportion of CBD) in the mixture may be selected from a range of, for example, about 10 or less (e.g., 8 or less), and may be 5 or less (e.g., 4 or less), preferably 3 or less (e.g., 2.8 or less), more preferably 2.5 or less (e.g., 2.2 or less), particularly 2 or less (e.g., 1.8 or less, 1.5 or less, 1.2 or less, 1 or less, 0.9 or less, 0.8 or less, 0.7 or less, 0.6 or less, 0.55 or less, 0.5 or less, 0.45 or less, 0.4 or less, 0.35 or less, 0.32 or less, 0.3 or less, 0.28 or less, 0.25 or less, or 0.22 or less), and may be 0.001 or more (e.g., 0.005 or more, 0.01 or more, 0.03 or more, 0.05 or more, 0.07 or more, 0.08 or more, 0.1 or more, 0.12 or more, 0.15 or more, 0.18 or more, or 0.2 or more).

When the mixture (a reaction mixture, etc.) comprises abn-CBD, the proportion (e.g., the proportion on an HPLC area basis) of abn-CBD relative to CBD (i.e., the proportion of abn-CBD/the proportion of CBD) in the mixture may be selected from a range of, for example, about 10 or less (e.g., 8 or less), and may be 5 or less (e.g., 4 or less), preferably 3 or less (e.g., 2.8 or less), more preferably 2.5 or less (e.g., 2.2 or less), particularly 2 or less (e.g., 1.8 or less, 1.5 or less, 1.2 or less, 1 or less, 0.9 or less, 0.8 or less, 0.7 or less, 0.6 or less, 0.55 or less, 0.5 or less, 0.45 or less, 0.4 or less, 0.35 or less, 0.32 or less, 0.3 or less, 0.28 or less, 0.25 or less, 0.22 or less, or 0.2 or less), and may be 0.001 or more (e.g., 0.005 or more, 0.01 or more, 0.03 or more, 0.05 or more, 0.07 or more, 0.08 or more, 0.1 or more, 0.12 or more, 0.15 or more, or 0.18 or more).

When the mixture (a reaction mixture, etc.) comprises olivetol and abn-CBD, the proportion (e.g., the proportion on an HPLC area basis) of olivetol and abn-CBD relative to CBD (i.e., the sum of the proportions of olivetol and abn-CBD/the proportion of CBD) in the mixture may be selected from a range of, for example, about 20 or less (e.g., 15 or less), and may be 10 or less (e.g., 8 or less), preferably 6 or less (e.g., 5.5 or less), more preferably 5 or less (e.g., 4.5 or less), particularly 4 or less (e.g., 3.5 or less, 3 or less, 2.5 or less, 2 or less, 1.8 or less, 1.6 or less, 1.5 or less, 1.2 or less, 1 or less, 0.9 or less, 0.8 or less, 0.7 or less, 0.6 or less, 0.55 or less, 0.5 or less, or 0.45 or less), and may be 0.002 or more (e.g., 0.01 or more, 0.02 or more, 0.05 or more, 0.1 or more, 0.15 or more, 0.18 or more, 0.2 or more, 0.25 or more, 0.3 or more, 0.35 or more, or 0.4 or more).

When the mixture (a reaction mixture, etc.) comprises the 2:1 adduct, the proportion (e.g., the proportion on an HPLC area basis) of the 2:1 adduct relative to CBD (i.e., the proportion of the 2:1 adduct/the proportion of CBD) in the mixture may be selected from a range of, for example, about 3 or less (e.g., 2.5 or less), and may be 2 or less (e.g., 1.5 or less), preferably 1 or less (e.g., 0.8 or less), more preferably 0.5 or less (e.g., 0.3 or less), particularly 0.2 or less (e.g., 0.18 or less, 0.15 or less, 0.12 or less, 0.1 or less, or 0.09 or less), and may be 0.0001 or more (e.g., 0.0005 or more, 0.001 or more, 0.005 or more, 0.01 or more, 0.02 or more, 0.03 or more, 0.04 or more, 0.05 or more, 0.06 or more, 0.07 or more, or 0.08 or more).

When the mixture (a reaction mixture, etc.) comprises abn-CBD and the 2:1 adduct, the proportion (e.g., the proportion on an HPLC area basis) of abn-CBD and the 2:1 adduct relative to CBD (i.e., the sum of the proportions of abn-CBD and the 2:1 adduct/the proportion of CBD) in the mixture may be selected from a range of, for example, about 15 or less (e.g., 12 or less), and may be 10 or less (e.g., 8 or less), preferably 5 or less (e.g., 4 or less), more preferably 3 or less (e.g., 2.8 or less), particularly 2.5 or less (e.g., 2.1 or less, 2 or less, 1.8 or less, 1.5 or less, 1.2 or less, 1 or less, 0.9 or less, 0.8 or less, 0.7 or less, 0.6 or less, 0.55 or less, 0.5 or less, 0.45 or less, 0.4 or less, 0.35 or less, 0.32 or less, or 0.3 or less), and may be 0.001 or more (e.g., 0.005 or more, 0.01 or more, 0.03 or more, 0.05 or more, 0.07 or more, 0.08 or more, 0.1 or more, 0.12 or more, 0.15 or more, 0.18 or more, 0.2 or more, 0.22 or more, 0.25 or more, or 0.28 or more).

When the mixture (a reaction mixture, etc.) comprises Δ9-THC, the proportion (e.g., the proportion on an HPLC area basis) of Δ9-THC relative to CBD (i.e., the proportion of Δ9-THC/the proportion of CBD) in the mixture may be selected from a range of, for example, about 1 or less (e.g., 0.8 or less), and may be 0.5 or less (e.g., 0.4 or less), preferably 0.3 or less (e.g., 0.28 or less), more preferably 0.25 or less (e.g., 0.22 or less), particularly 0.2 or less (e.g., 0.18 or less, 0.15 or less, 0.12 or less, 0.1 or less, 0.09 or less, 0.08 or less, 0.07 or less, 0.06 or less, 0.055 or less, 0.05 or less, 0.045 or less, 0.04 or less, or 0.035 or less), and may be 0.0001 or more (e.g., 0.0005 or more, 0.001 or more, 0.003 or more, 0.005 or more, 0.007 or more, 0.008 or more, 0.01 or more, 0.012 or more, 0.015 or more, 0.018 or more, 0.02 or more, 0.022 or more, 0.025 or more, 0.028 or more, or 0.03 or more).

When the mixture (a reaction mixture, etc.) comprises Δ8-THC, the proportion (e.g., the proportion on an HPLC area basis) of Δ8-THC relative to CBD (i.e., the proportion of Δ8-THC/the proportion of CBD) in the mixture may be selected from a range of, for example, about 0.1 or less (e.g., 0.08 or less), and may be 0.05 or less (e.g., 0.04 or less), preferably 0.03 or less (e.g., 0.028 or less), more preferably 0.025 or less (e.g., 0.022 or less), particularly 0.02 or less (e.g., 0.018 or less, 0.015 or less, 0.012 or less, 0.01 or less, 0.009 or less, 0.008 or less, 0.007 or less, 0.006 or less, 0.0055 or less, 0.005 or less, 0.0045 or less, or 0.004 or less), and may be 0.00001 or more (e.g., 0.00005 or more, 0.0001 or more, 0.0003 or more, 0.0005 or more, 0.0007 or more, 0.0008 or more, 0.001 or more, 0.0012 or more, 0.0015 or more, 0.0018 or more, 0.002 or more, 0.0022 or more, 0.0025 or more, 0.0028 or more, or 0.003 or more).

When the mixture (a reaction mixture, etc.) comprises Δ9-THC and Δ8-THC, the proportion (e.g., the proportion on an HPLC area basis) of Δ9-THC and Δ8-THC relative to CBD [i.e., (the sum of the proportions of Δ9-THC and Δ8-THC)/the proportion of CBD] in the mixture may be selected from a range of, for example, about 1 or less (e.g., 0.8 or less), and may be 0.5 or less (e.g., 0.4 or less), preferably 0.3 or less (e.g., 0.28 or less), more preferably 0.25 or less (e.g., 0.22 or less), particularly 0.2 or less (e.g., 0.18 or less, 0.15 or less, 0.12 or less, 0.1 or less, 0.09 or less, 0.08 or less, 0.07 or less, 0.06 or less, 0.055 or less, 0.05 or less, 0.045 or less, or 0.04 or less), and may be 0.0001 or more (e.g., 0.0005 or more, 0.001 or more, 0.003 or more, 0.005 or more, 0.007 or more, 0.008 or more, 0.01 or more, 0.012 or more, 0.015 or more, 0.018 or more, 0.02 or more, 0.022 or more, 0.025 or more, 0.028 or more, 0.03 or more, 0.032 or more, or 0.035 or more).

In the mixture (a reaction mixture, etc.), the proportion (e.g., the proportion on an HPLC area basis) of olivetol, abn-CBD, the 2:1 adduct, Δ9-THC, and Δ8-THC relative to CBD [i.e., (the sum of the proportions of olivetol, abn-CBD, the 2:1 adduct, Δ9-THC, and Δ8-THC)/the proportion of CBD] in the mixture may be selected from a range of, for example, about 30 or less (e.g., 25 or less), and may be 20 or less (e.g., 15 or less), preferably 10 or less (e.g., 8 or less), more preferably 5 or less (e.g., 4 or less), particularly 3 or less (e.g., 2.8 or less, 2.5 or less, 2.2 or less, 2 or less, 1.9 or less, 1.8 or less, 1.7 or less, 1.6 or less, 1.5 or less, 1.4 or less, 1.3 or less, 1.2 or less, 1.1 or less, 1 or less, less than 1, 0.95 or less, 0.9 or less, 0.85 or less, 0.8 or less, or 0.75 or less), and may be 0.001 or more (e.g., 0.01 or more, 0.03 or more, 0.05 or more, 0.1 or more, 0.15 or more, 0.2 or more, 0.25 or more, 0.3 or more, 0.35 or more, 0.4 or more, 0.45 or more, 0.5 or more, 0.55 or more, 0.6 or more, 0.65 or more, or 0.7 or more).

In the mixture (a reaction mixture, etc.), the proportion (e.g., the proportion on an HPLC area basis) of abn-CBD, the 2:1 adduct, Δ9-THC, and Δ8-THC relative to CBD [i.e., (the sum of the proportions of abn-CBD, the 2:1 adduct, Δ9-THC, and Δ8-THC)/the proportion of CBD] may be selected from a range of, for example, about 20 or less (e.g., 15 or less), and may be 10 or less (e.g., 8 or less), preferably 5 or less (e.g., 4 or less), more preferably 3 or less (e.g., 2.5 or less), particularly 2 or less (e.g., 1.9 or less, 1.8 or less, 1.7 or less, 1.6 or less, 1.5 or less, 1.4 or less, 1.3 or less, 1.2 or less, 1.1 or less, 1 or less, less than 1, 0.95 or less, 0.9 or less, 0.85 or less, 0.8 or less, 0.75 or less, 0.7 or less, 0.65 or less, 0.6 or less, or 0.55 or less), and may be 0.001 or more (e.g., 0.005 or more, 0.01 or more, 0.02 or more, 0.05 or more, 0.1 or more, 0.15 or more, 0.2 or more, 0.25 or more, 0.3 or more, 0.35 or more, 0.4 or more, 0.45 or more, or 0.5 or more).

### First Purified Product (Crude CBD)

As described above, the reaction mixture (mixture after the reaction) generally contains components other than CBD.

Accordingly, such a mixture may be further subjected to purification [CBD may be purified from the reaction mixture (components other than CBD (e.g., at least by-products) may be separated)].

The purification method (CBD purification (separation) method, purification technique, purification procedure) is not particularly limited, and conventional methods (e.g., extraction, crystallization, recrystallization, distillation, chromatography, filtration, concentration) may be used.

In this regard, in the above-described mixture (a reaction mixture), it is also possible to relatively suppress the generation of by-products [and it is also possible to obtain a mixture containing a relatively small amount of by-products (at a smaller amount relative to CBD)]. Therefore, by using such a mixture (e.g., a reaction mixture obtained by the method according to the present invention), efficient purification can be achieved in combination with the formation of a solvate, as described above.

The present inventors have found a method that achieves more efficient purification even when using such a mixture [a mixture in which the generation of by-products is relatively suppressed; a mixture containing a relatively small amount of by-products (at a smaller amount relative to CBD)]. The method is described in detail below.

### Adsorption Step (First Purification Step)

First, the mixture is subjected to adsorption treatment, as necessary.

Such adsorption treatment appears to allow relatively efficient separation [selective separation (separation by adsorption)] of by-products, in particular, olivetol and abn-CBD. Therefore, such adsorption treatment is particularly effective for mixtures comprising (particularly those with a relatively large amount of) olivetol and/or abn-CBD (at least one component selected from the group consisting of olivetol and abn-CBD).

Among them, according to studies conducted by the present inventors, it appears preferable to separate olivetol to a high degree (as much as possible) because olivetol is a highly polar component and tends to interfere with the crystal formation of CBD, among other reasons. Thus, olivetol can make purification of CBD difficult or complicated. In particular, such adsorption treatment can be suitably carried out for mixtures comprising at least olivetol (in particular a mixtures comprising olivetol in a relatively large proportion).

The mixture (mixture to be subjected to adsorption treatment) may be a mixture containing a solvent (a solvent mixture), such as the reaction mixture obtained by the method described above.

Examples of the solvent include hydrocarbons [e.g., aliphatic hydrocarbons (such as pentane, hexane, isohexane, heptane, 2-methylhexane, 2,2-dimethylpentane, octane, isooctane, nonane, decane, cyclopentane, cyclohexane, cycloheptane, and methylcyclohexane,), aromatic hydrocarbons (such as benzene, toluene, xylene, and ethylbenzene)], halogenated solvents (e.g., halogenated hydrocarbons such as dichloromethane, chloroform, and carbon tetrachloride), ketones (e.g., acetone, methyl ethyl ketone, methyl isobutyl ketone, etc.), esters (e.g., aliphatic esters such as methyl acetate, ethyl acetate, butyl acetate, etc.), carbonates (e.g., ethylene carbonate, propylene carbonate, etc.), ethers [e.g., chain ethers (such as diethyl ether and methyl tert-butyl ether), cyclic ethers (such as tetrahydrofuran and dioxane) etc.], glycol ethers (e.g., ethylene glycol monomethyl ether, ethylene glycol monoethyl ether, propylene glycol monomethyl ether, diethylene glycol monoethyl ether, etc.), glycol ether esters (e.g., ethylene glycol monomethyl ether acetate, etc.), nitrogen-containing solvents {e.g., amides [such as N-substituted chain amides (e.g., N-alkyl-substituted alkanamides such as N,N-dimethylformamide), cyclic amides (e.g., 2-pyrrolidone), and the like], etc.}, nitriles (e.g., acetonitrile, etc.), sulfur-containing solvents (e.g., sulfoxides such as dimethyl sulfoxide; sulfones such as dimethyl sulfone and sulfolane), alcohols [e.g., alkanols (such as methanol, ethanol, and isopropanol)], polyols (e.g., alkanediols such as ethylene glycol and propylene glycol; glycerol etc.), water, and the like.

The solvent may be used singly or in combination of two or more.

Among these solvents, from the viewpoints of, for example, efficient separation (separation by adsorption treatment) of by-products (e.g., olivetol, abn-CBD, etc., in particular at least olivetol), aprotic solvents (e.g., low-polarity aprotic solvents) and the like may be suitably used, and hydrocarbons (in particular aliphatic hydrocarbons) may be especially suitably used.

Therefore, the solvent may comprise at least an aprotic solvent and/or a hydrocarbon, and may particularly comprise at least an aliphatic hydrocarbon {e.g., aliphatic hydrocarbons [e.g., aliphatic hydrocarbons (e.g., chain-structured or cyclic aliphatic hydrocarbons, saturated or unsaturated hydrocarbons, chain-structured or cyclic saturated aliphatic hydrocarbons, etc.) having 5 or more carbon atoms (e.g., 5 to 18 carbon atoms, 6 to 12 carbon atoms, 6 to 10 carbon atoms, etc.), etc.] such as pentane, hexane, isohexane, heptane, 2-methylhexane, 2,2-dimethylpentane, octane, isooctane, nonane, decane, cyclopentane, cyclohexane, cycloheptane, and methylcyclohexane, and the like}.

When the solvent contains an aliphatic hydrocarbon, the proportion of the aliphatic hydrocarbon (e.g., heptane, etc.) relative to the total amount of solvent may be selected from a range of, for example, about 1 vol% or more (e.g., 3 vol% or more), preferably about 5 vol% or more (e.g., 10 vol% or more), more preferably about 15 vol% or more (e.g., 20 vol% or more), and may be 25 vol% or more [e.g., 30 vol% or more, 35 vol% or more, 40 vol% or more, 50 vol% or more, greater than 50 vol%, 60 vol% or more, 70 vol% or more, 80 vol% or more, 85 vol% or more, 90 vol% or more, 95 vol% or more, or (substantially) 100 vol%, or the solvent consisting of (consisting essentially of) an aliphatic hydrocarbon].

The aliphatic hydrocarbon may be used in combination with other (additional) solvent (a solvent other than an aliphatic hydrocarbon, e.g., an aromatic hydrocarbon such as toluene). In such a case, the proportion of the other solvent in the total amount of solvent may be, for example, about 99 vol% or less (e.g., 97 vol% or less), preferably about 95 vol% or less (e.g., 90 vol% or less), more preferably about 85 vol% or less (e.g., 80 vol% or less, 75 vol% or less, 70 vol% or less, or 65 vol% or less), and may be 1 vol% or more (e.g., 3 vol% or more, 5 vol% or more, 10 vol% or more, 15 vol% or more, 20 vol% or more, 25 vol% or more, 30 vol% or more, or 35 vol% or more).

The solvent may or may not contain a protic solvent (e.g., water, alcohols, and the like). From the viewpoints of, for example, efficient separation (separation by adsorption treatment) of by-products (e.g., olivetol, abn-CBD, in particular at least olivetol), the solvent preferably is free from or contains a protic solvent only in a small amount [e.g., the amount of protic solvent being 10 vol% or less (e.g., 5 vol% or less, 3 vol% or less, 1 vol% or less, 0.5 vol% or less, or 0.1 vol% or less) in the solvent]. In particular, the solvent may be free from or contain water only in a small amount [e.g., the amount of water being 10 vol% or less of the solvent (e.g., 5 vol% or less, 3 vol% or less, 1 vol% or less, 0.5 vol% or less, or 0.1 vol% or less) in the solvent].

In the mixture containing a solvent, the proportion (concentration) of components (solid components) other than the solvent relative to the total amount of the solvent and components other than the solvent may be, for example, about 0.1 mass% or more (e.g., 0.5 mass% or more, 1 mass% or more, 3 mass% or more, or 5 mass% or more), and may be 99 mass% or less (e.g., 95 mass% or less, 90 mass% or less, 80 mass% or less, 70 mass% or less, 60 mass% or less, 50 mass% or less, 45 mass% or less, 40 mass% or less, 35 mass% or less, 30 mass% or less, 25 mass% or less, 20 mass% or less, 15 mass% or less, 12 mass% or less, or 10 mass% or less).

In the mixture containing a solvent, the amount of the solvent relative to 1 part of CBD (by volume) may be about 0.01 or more (e.g., 0.05 or more, 0.1 or more, 0.3 or more, or 0.5 or more), and may be 1000 or less (e.g., 800 or less, 500 or less, 300 or less, 200 or less, 150 or less, 120 or less, 100 or less, 80 or less, 70 or less, 60 or less, 50 or less, or 40 or less).

The mixture containing a solvent may be prepared by an appropriate method depending on factors such as the reaction mixture used and the type and amount of the solvent. For example, when using a reaction mixture from which the reaction solvent (the solvent used in the reaction) has been separated (removed), a solvent may be added to prepare a mixture. Alternatively, when using a reaction mixture containing a reaction solvent, in the case where the reaction solvent and an adsorption solvent (a solvent used for adsorption treatment) are different, after separating the reaction solvent (e.g., by concentration or the like), the adsorption solvent may be mixed therewith; in the case where both solvents are the same solvent, the reaction solvent may be used as the adsorption solvent as it is; and in the case where both solvents are the same but differ in the amount (proportion), the amount of the solvent may be adjusted as appropriate (e.g., by concentration or mixing).

The adsorption treatment method is not particularly limited, and typically an adsorbent (adsorption material) may be brought into contact with a mixture (in particular, a solvent-containing mixture).

Examples of the adsorbent include silica (silica gel), alumina (activated alumina), zeolite, Celite, diatomaceous earth, clay, activated clay, activated carbon, synthetic adsorbents, ion-exchange resins (e.g., activated alumina and ion-exchange resin), and the like.

Among these, silica may be suitably used from the viewpoints of, for example, efficient separation (selective separation) of by-products (e.g., olivetol, abn-CBD, etc., in particular at least olivetol).

The shape of the adsorbent (silica, etc.) is not particularly limited, and may include particulate forms, which also encompass spherical forms and the like.

When the adsorbent is particulate, the particle size is not particularly limited and may be, for example, an average particle size of 1 to 500 µm (e.g., 3 to 300 µm, 5 to 100 µm).

The adsorbent may be used singly or in combination of two or more.

The amount (usage amount) of the adsorbent may be selected depending on factors such as the adsorption treatment method (mode of adsorption treatment), the type (adsorption capacity) of adsorbent, and the amount of by-products, and is not particularly limited. For example, relative to 1 part by mass of CBD [or the solid components (components other than the solvent) contained in the mixture], the amount of adsorbent may be selected from a range of about 0.01 parts by mass or more (e.g., 0.05 parts by mass or more, 0.1 parts by mass or more, 0.15 parts by mass or more), and may be 0.2 parts by mass or more (e.g., 0.25 parts by mass or more), preferably 0.3 parts by mass or more (e.g., 0.35 parts by mass or more), more preferably 0.4 parts by mass or more (e.g., 0.45 parts by mass or more, or 0.5 parts by mass or more).

The amount (upper limit) of the adsorbent is not particularly limited. For example, relative to 1 part by mass of CBD [or the solid components (components other than the solvent) contained in the mixture], the amount may be selected from a range of about 300 parts by mass or less (e.g., 100 parts by mass or less) and may be 80 parts by mass or less (e.g., 50 parts by mass or less), preferably 30 parts by mass or less (e.g., 20 parts by mass or less), and more preferably 10 parts by mass or less (e.g., 8 parts by mass or less, 5 parts by mass or less, 3 parts by mass or less, 2 parts by mass or less, or 1 part by mass or less).

The adsorption treatment method (method of contacting the adsorbent with the mixture) is not particularly limited, and may be appropriately selected depending on factors such as the form of the adsorbent. Examples thereof include a method of mixing the adsorbent with the mixture, a method of passing the mixture through the adsorbent [e.g., a method of passing the mixture through an adsorbent fixed by appropriate means (e.g., a column packed with the adsorbent)], and a method combining these methods.

Among these, from the viewpoints of, for example, efficient separation of by-products (e.g., olivetol, abn-CBD, in particular at least olivetol), a method of mixing the adsorbent with the mixture may be suitably used.

The mixing may be carried out under stirring.

The mixing may also be carried out under an inert atmosphere (e.g., nitrogen, argon), and the like.

The vessel used in the mixing is not particularly limited, and conventional vessels may be used.

The adsorption treatment conditions are not particularly limited, and may be appropriately selected as appropriate depending on factors such as the amount and type of the adsorbent.

For example, the adsorption may be carried out at room temperature (or ambient temperature), under heating, or under cooling. Specific adsorption temperatures include, for example, -30 to 80°C (e.g., 0 to 60°C, 5 to 50°C, 10 to 40°C). The adsorption treatment time (e.g., the time during which the adsorbent is brought into contact with the mixture) may be selected depending on factors such as the type of adsorbent and the mode of the adsorption method, and is not particularly limited; the adsorption treatment time may be, for example, about 1 minute or more (e.g., 3 minutes or more), preferably 5 minutes or more (e.g., 10 minutes or less), more preferably 20 minutes or more (e.g., 30 minutes or more), and may be 24 hours or less (e.g., 12 hours or less, 6 hours or less, or 3 hours or less).

After the adsorption treatment, depending on the adsorption treatment (method) and the like, the adsorbent may be separated (e.g., the adsorbent may be separated from the mixture containing the adsorbent). The separation method may be appropriately selected depending on the adsorbent and the mode of the adsorption treatment, and examples include filtration, decantation, and the like.

The adsorbent may be washed (washing treatment) if necessary, and the washing liquid [or solid components or solid fraction (at least CBD, etc.) recovered from the washing liquid (e.g., recovered by concentration, etc.)] may be mixed with the mixture after the adsorption treatment. By such a method (washing step), CBD and the like can also be recovered from the adsorbent [e.g., CBD, etc. adhering to the adsorbent (adhering to the adsorbent without being adsorbed)]. Therefore, after the adsorption treatment, such a washing step [a step of washing the adsorbent (washing the adsorbent to recover CBD and the like)] may further be carried out.

The washing solvent is not particularly limited, and the solvents exemplified above may be used. Preferred embodiments of the solvent are also the same as above; for example, hydrocarbons (in particular aliphatic hydrocarbons), preferably solvents comprising at least an aliphatic hydrocarbon (e.g., an aliphatic hydrocarbon, or an aliphatic hydrocarbon in combination with an aromatic hydrocarbon), may be used.

The amount of the washing solvent used may be appropriately selected depending on factors such as the amount of the adsorbent.

After the adsorption treatment (optionally, further the washing treatment), if necessary, the solvent (or a solvent component, e.g., an adsorption solvent, a washing solvent, etc.) may be separated (e.g., separated by concentration, etc.).

Through the above-described steps [adsorption treatment (adsorption treatment step) and, optionally, further a washing step, other steps as needed], it is possible to obtain a purified product comprising CBD [sometimes referred to as a first purified product, a crude product, or the like (as compared with purified products described later)] {it is possible to purify the mixture [by separating (selectively separating) components other than CBD (in particular at least one component selected from the group consisting of olivetol and abn-CBD) from the mixture]}.

It should be noted that CBD can be substantially completely recovered even after the adsorption treatment or the like. For example, when A1 denotes the mass of CBD in the mixture (the mixture subjected to the adsorption treatment) and A2 denotes the mass of CBD in the first purified product, the value of "A2/A1" may be selected from a range of, for example, about 0.5 or more (e.g., 0.6 or more), and may be 0.7 or more (e.g., 0.75 or more, 0.8 or more, 0.85 or more, 0.88 or more, 0.9 or more, 0.92 or more, 0.95 or more, 0.96 or more, 0.97 or more, 0.98 or more, 0.99 or more, or substantially 1).

In the first purified product, the proportion (e.g., the proportion on an HPLC area (peak area) basis) of CBD relative to the total amount of CBD, olivetol, abn-CBD, the 2:1 adduct, Δ9-THC, and Δ8-THC may be selected from a range of, for example, 40% or more (e.g., 45% or more), and may be 50% or more (e.g., 52% or more), preferably 55% or more (e.g., 58% or more), more preferably 60% or more (e.g., 62% or more), particularly 65% or more (e.g., 66% or more, 67% or more, 68% or more, 69% or more, 70% or more, 71% or more, 72% or more, 73% or more, 74% or more, 75% or more, 76% or more, 77% or more, 78% or more, 79% or more, or 80% or more), and may be 99.9% or less (e.g., 99.5% or less, 99% or less, 98% or less, 97% or less, 95% or less, 92% or less, 90% or less, 88% or less, 85% or less, 82% or less, or 80% or less).

When M1 denotes the proportion (e.g., the proportion on an HPLC area (peak area) basis) of CBD relative to the total amount of CBD, olivetol, abn-CBD, the 2:1 adduct, Δ9-THC, and Δ8-THC in the mixture, and M2 denotes the proportion (e.g., the proportion on an HPLC area (peak area) basis) of CBD relative to the total amount of CBD, olivetol, abn-CBD, the 2:1 adduct, Δ9-THC, and Δ8-THC in the first purified product, the value of "M2 - M1" may be greater than 0%, and may be selected from a range of 0.1% or more (e.g., 0.5% or more), and may be 1% or more (e.g., 1.5% or more), preferably 2% or more (e.g., 2.5% or more), more preferably 3% or more (e.g., 4% or more), and particularly 5% or more (e.g., 7% or more, 8% or more, 10% or more, 12% or more, or 15% or more).

The first purified product may or may not contain olivetol. Even when olivetol is present, olivetol is often present only in a small amount. For example, in the first purified product, the proportion of olivetol relative to the total amount of CBD, olivetol, abn-CBD, the 2:1 adduct, Δ9-THC, and Δ8-THC (e.g., the proportion on an HPLC area basis) may be, for example, 20% or less (e.g., 15% or less, 12% or less, 10% or less, 8% or less, 5% or less, 3% or less, 2% or less, 1.5% or less, 1.2% or less, 1% or less, 0.9% or less, 0.8% or less, 0.7% or less, 0.6% or less, or 0.5% or less).

When the first purified product contains olivetol, the proportion (the lower limit of the proportion, e.g., the proportion on an HPLC area basis) of olivetol relative to the total amount of CBD, olivetol, abn-CBD, the 2:1 adduct, Δ9-THC, and Δ8-THC in the first purified product may be 0.0001%, 0.01%, 0.05%, 0.1%, 0.15%, 0.2%, 0.25%, 0.3%, 0.35%, 0.4%, and the like.

When M1 denotes the proportion (e.g., the proportion on an HPLC area (peak area) basis) of olivetol relative to the total amount of CBD, olivetol, abn-CBD, the 2:1 adduct, Δ9-THC, and Δ8-THC in the mixture (e.g., a mixture subjected to adsorption treatment or a reaction mixture), and M2 denotes the proportion (e.g., the proportion on an HPLC area (peak area) basis) of olivetol relative to the total amount of CBD, olivetol, abn-CBD, the 2:1 adduct, Δ9-THC, and Δ8-THC in the first purified product), the value of "M1 - M2" may be greater than 0%, may be selected from a range of about 0.01% or more (e.g., 0.1% or more, 0.3% or more, 0.5% or more), may be 1% or more (e.g., 1.5% or more), preferably 2% or more (e.g., 2.5% or more), and more preferably 3% or more (e.g., 3.5% or more, 4% or more, 5% or more, 6% or more, 7% or more, or 8% or more).

The first purified product may be free from or may contain abn-CBD. Even when abn-CBD is present, abn-CBD is often present only in a small amount. For example, in the first purified product, the proportion (e.g., the proportion on an HPLC area basis) of abn-CBD relative to the total amount of CBD, olivetol, abn-CBD, the 2:1 adduct, Δ9-THC, and Δ8-THC may be, for example, 30% or less (e.g., 25% or less, 20% or less, 15% or less, 12% or less, 10% or less, 9% or less, 8% or less, 7% or less, 6% or less, or 5% or less).

When the first purified product contains abn-CBD, the proportion (the lower limit of the proportion, e.g., the proportion on an HPLC area basis) of abn-CBD relative to the total amount of CBD, olivetol, abn-CBD, the 2:1 adduct, Δ9-THC, and Δ8-THC in the first purified product may be 0.01%, 0.1%, 0.5%, 1%, 1.5%, 2%, 2.5%, 3%, 3.5%, 4%, 4.5%, and the like.

When M1 denotes the proportion (e.g., the proportion on an HPLC area (peak area) basis) of abn-CBD relative to the total amount of CBD, olivetol, abn-CBD, the 2:1 adduct, Δ9-THC, and Δ8-THC in the mixture, and M2 denotes the proportion (e.g., the proportion on an HPLC area (peak area) basis) of abn-CBD relative to the total amount of CBD, olivetol, abn-CBD, the 2:1 adduct, Δ9-THC, and Δ8-THC in the first purified product, the value of "M1 - M2" may be greater than 0%, may be selected from a range of about 0.01% or more (e.g., 0.05% or more), may be 0.1% or more (e.g., 0.2% or more), preferably 0.3% or more (e.g., 0.5% or more), more preferably 1% or more (e.g., 1.5% or more), and particularly 2% or more (e.g., 3% or more, 3.5% or more, 4% or more, 4.5% or more, 5% or more, 5.5% or more, 6% or more, 6.5% or more, 7% or more, or 7.5% or more).

The first purified product may be free from olivetol and abn-CBD or may contain olivetol and abn-CBD. Even when olivetol and abn-CBD are present, they are often present only in small amounts. For example, in the first purified product, the proportion (e.g., the proportion on an HPLC area basis) of olivetol and abn-CBD relative to the total amount of CBD, olivetol, abn-CBD, the 2:1 adduct, Δ9-THC, and Δ8-THC may be, for example, 40% or less (e.g., 35% or less, 30% or less, 25% or less, 20% or less, 15% or less, 12% or less, 10% or less, 9% or less, 8% or less, 7% or less, 6% or less, or 5.5% or less).

When the first purified product contains olivetol and abn-CBD, the proportion (the lower limit of the proportion, e.g., the proportion on an HPLC area basis) of olivetol and abn-CBD relative to the total amount of CBD, olivetol, abn-CBD, the 2:1 adduct, Δ9-THC, and Δ8-THC in the first purified product may be 0.01%, 0.1%, 0.5%, 1%, 1.5%, 2%, 2.5%, 3%, 3.5%, 4%, 4.5%, 5%, and the like.

When M1 denotes the proportion (e.g., the proportion on an HPLC area (peak area) basis) of olivetol and abn-CBD relative to the total amount of CBD, olivetol, abn-CBD, the 2:1 adduct, Δ9-THC, and Δ8-THC in the mixture, and M2 denotes the proportion (e.g., the proportion on an HPLC area (peak area) basis) of olivetol and abn-CBD relative to the total amount of CBD, olivetol, abn-CBD, the 2:1 adduct, Δ9-THC, and Δ8-THC in the first purified product, the value of "M1 - M2" may be greater than 0%, may be selected from a range of about 0.05% or more (e.g., 0.1% or more), may be 0.5% or more (e.g., 1% or more), preferably 2% or more (e.g., 3% or more), more preferably 5% or more (e.g., 7% or more), and particularly 8% or more (e.g., 9% or more, 10% or more, 11% or more, 12% or more, 13% or more, 14% or more, 15% or more, 16% or more, 17% or more, 18% or more, 19% or more, or 20% or more).

The first purified product may contain other components (e.g., by-products).

For example, when the first purified product contains the 2:1 adduct, the proportion (e.g., the proportion on an HPLC area basis) of the 2:1 adduct relative to the total amount of CBD, olivetol, abn-CBD, the 2:1 adduct, Δ9-THC, and Δ8-THC in the first purified product may be selected from a range of, for example, 0.01% or more (e.g., 0.05% or more), and may be 0.1% or more (e.g., 0.3% or more), preferably 0.5% or more (e.g., 0.7% or more), more preferably 1% or more (e.g., 1.2% or more), particularly 1.5% or more (e.g., 1.8% or more, 2% or more, 2.2% or more, 2.5% or more, 2.8% or more, 3% or more, 3.2% or more, 3.5% or more, 3.8% or more, 4% or more, 4.2% or more, 4.5% or more, 4.8% or more, 5% or more, 5.2% or more, 5.5% or more, 5.8% or more, 6% or more, 7.2% or more, or 7.5% or more), and may be 50% or less (e.g., 45% or less, 40% or less, 35% or less, 30% or less, 25% or less, 22% or less, 20% or less, 18% or less, 15% or less, 12% or less, 10% or less, 9% or less, 8% or less, or 7% or less).

When the first purified product contains Δ9-THC, the proportion (e.g., the proportion on an HPLC area basis) of Δ9-THC relative to the total amount of CBD, olivetol, abn-CBD, the 2:1 adduct, Δ9-THC, and Δ8-THC in the first purified product may be selected from a range of, for example, 0.01% or more (e.g., 0.03% or more), and may be 0.05% or more (e.g., 0.1% or more), preferably 0.2% or more (e.g., 0.3% or more), more preferably 0.5% or more (e.g., 0.7% or more), and particularly 0.8% or more (e.g., 1% or more, 1.2% or more, 1.3% or more, 1.4% or more, 1.5% or more, 1.6% or more, 1.7% or more, 1.8% or more, 1.9% or more, 2% or more, 2.1% or more, 2.2% or more, 2.3% or more, 2.4% or more, 2.5% or more, 2.6% or more, 2.7% or more, or 2.8% or more), and may be 20% or less (e.g., 18% or less, 15% or less, 12% or less, 10% or less, 9% or less, 8% or less, 7% or less, 6% or less, 5% or less, 4.5% or less, 4% or less, 3.5% or less, or 3% or less).

When the first purified product contains Δ8-THC, the proportion (e.g., the proportion on an HPLC area basis) of Δ8-THC relative to the total amount of CBD, olivetol, abn-CBD, the 2:1 adduct, Δ9-THC, and Δ8-THC in the first purified product may be selected from a range of, for example, 0.001% or more (e.g., 0.005% or more), and may be 0.01% or more (e.g., 0.03% or more), preferably 0.05% or more (e.g., 0.07% or more), more preferably 0.1% or more (e.g., 0.12% or more), particularly 0.15% or more (e.g., 0.2% or more, 0.25% or more, 0.3% or more, 0.35% or more, 0.4% or more, 0.45% or more, 0.5% or more, 0.55% or more, 0.6% or more, 0.65% or more, or 0.7% or more), and may be 10% or less (e.g., 8% or less, 5% or less, 4% or less, 3% or less, 2% or less, 1.5% or less, 1% or less, 0.9% or less, or 0.8% or less).

When the first purified product contains Δ9-THC and Δ8-THC, the proportion (e.g., the proportion on an HPLC area) of Δ9-THC and Δ8-THC relative to the total amount of CBD, olivetol, abn-CBD, the 2:1 adduct, Δ9-THC, and Δ8-THC in the first purified product may be selected from a range of, for example, 0.01% or more (e.g., 0.03% or more), and may be 0.05% or more (e.g., 0.1% or more), preferably 0.2% or more (e.g., 0.3% or more), more preferably 0.5% or more (e.g., 0.7% or more), and particularly 0.8% or more (e.g., 1% or more, 1.2% or more, 1.3% or more, 1.4% or more, 1.5% or more, 1.6% or more, 1.7% or more, 1.8% or more, 1.9% or more, 2% or more, 2.1% or more, 2.2% or more, 2.3% or more, 2.4% or more, 2.5% or more, 2.6% or more, 2.7% or more, 2.8% or more, 2.9% or more, 3% or more, 3.1% or more, 3.2% or more, 3.3% or more, 3.4% or more, 3.5% or more, or 3.6% or more), and may be 20% or less (e.g., 18% or less, 15% or less, 12% or less, 10% or less, 9% or less, 8% or less, 7% or less, 6% or less, 5.5% or less, 5% or less, 4.5% or less, or 4% or less).

When the first purified product contains olivetol, the ratio of the proportion (e.g., the proportion on an HPLC area) of olivetol relative to the proportion of CBD (i.e., the proportion of olivetol/the proportion of CBD) in the first purified product may be selected from a range of, for example, about 1 or less (e.g., 0.5 or less), and may be 0.3 or less (e.g., 0.25 or less), preferably 0.2 or less (e.g., 0.15 or less), more preferably 0.1 or less (e.g., 0.05 or less), and particularly 0.03 or less (e.g., 0.02 or less, 0.01 or less, 0.009 or less, 0.008 or less, 0.007 or less, or 0.006 or less), and may be 0.00001 or more (e.g., 0.00005 or more, 0.0001 or more, 0.0005 or more, 0.001 or more, 0.002 or more, 0.003 or more, 0.004 or more, or 0.005 or more).

When N1 denotes the ratio of the proportion (e.g., the proportion on an HPLC area (peak area) basis) of olivetol relative to the proportion of CBD (i.e., the proportion of olivetol/the proportion of CBD) in the mixture, and N2 denotes the ratio of the proportion (e.g., the proportion on an HPLC area (peak area) basis) of olivetol relative to the proportion of CBD (i.e., the ratio of the proportion of olivetol/the proportion of CBD) in the first purified product, the value of "N1 - N2" may be greater than 0, and may be selected from a range of about 0.01 or more (e.g., 0.02 or more), and may be 0.03 or more (e.g., 0.05 or more), preferably 0.07 or more (e.g., 0.08 or more), more preferably 0.1 or more (e.g., 0.12 or more or 0.15 or more).

When the first purified product contains abn-CBD, the ratio of the proportion (e.g., the proportion on an HPLC area basis) of abn-CBD relative to the proportion of CBD (i.e., the proportion of abn-CBD/the proportion of CBD) in the first purified product may be selected from a range of, for example, about 10 or less (e.g., 5 or less), and may be 3 or less (e.g., 2.5 or less), preferably 2 or less (e.g., 1.5 or less), more preferably 1 or less (e.g., 0.5 or less), and particularly 0.3 or less (e.g., 0.2 or less, 0.1 or less, 0.09 or less, 0.08 or less, 0.07 or less, or 0.06 or less), and may be 0.0001 or more (e.g., 0.0005 or more, 0.001 or more, 0.005 or more, 0.01 or more, 0.02 or more, 0.03 or more, 0.04 or more, or 0.05 or more).

When N1 denotes the ratio of the proportion (e.g., the proportion on an HPLC area (peak area) basis) of abn-CBD relative to the proportion of CBD (i.e., the proportion of abn-CBD/the proportion of CBD) in the mixture, and N2 denotes the ratio of the proportion (e.g., the proportion on an HPLC area (peak area) basis) of abn-CBD relative to the proportion of CBD (i.e., the proportion of abn-CBD/the proportion of CBD) in the first purified product, the value of "N1 - N2" may be greater than 0, and may be selected from a range of about 0.01 or more (e.g., 0.02 or more), and may be 0.03 or more (e.g., 0.05 or more), preferably 0.07 or more (e.g., 0.08 or more), more preferably 0.1 or more (e.g., 0.12 or more or 0.15 or more).

When the first purified product contains olivetol and abn-CBD, the ratio of the proportion (e.g., the proportion on an HPLC area basis) of olivetol and abn-CBD relative to the proportion of CBD [i.e., (the proportion of olivetol and abn-CBD)/the proportion of CBD] in the first purified product may be selected from a range of, for example, about 10 or less (e.g., 7 or less), and may be 5 or less (e.g., 3 or less), preferably 2.5 or less (e.g., 2 or less), more preferably 1.5 or less (e.g., 1 or less), and particularly 0.5 or less (e.g., 0.4 or less, 0.3 or less, 0.2 or less, 0.1 or less, 0.09 or less, 0.08 or less, or 0.07 or less), and may be 0.0001 or more (e.g., 0.0005 or more, 0.001 or more, 0.005 or more, 0.01 or more, 0.02 or more, 0.03 or more, 0.04 or more, 0.05 or more, or 0.06 or more).

When N1 denotes the ratio of the proportion (e.g., the proportion on an HPLC area (peak area) basis) of olivetol and abn-CBD relative to the proportion of CBD (i.e., the sum of the proportions of olivetol and abn-CBD/the proportion of CBD) in the mixture and N2 denotes the ratio of the proportion (e.g., the proportion on an HPLC area (peak area) basis) of olivetol and abn-CBD relative to the proportion of CBD (i.e., the sum of the proportions of olivetol and abn-CBD/the proportion of CBD) in the first purified product, the value of "N1 - N2" may be greater than 0, and may be selected from a range of about0.01 or more (e.g., 0.03 or more), and may be 0.05 or more (e.g., 0.07 or more), preferably 0.1 or more (e.g., 0.15 or more), more preferably 0.2 or more (e.g., 0.25 or more, 0.3 or more).

When the first purified product contains the 2:1 adduct, the ratio of the proportion (e.g., the proportion on an HPLC area basis) of the 2:1 adduct relative to the proportion of CBD (i.e., the proportion of the 2:1 adduct/the proportion of CBD) in the first purified product may be selected from a range of, for example, about 3 or less (e.g., 2.5 or less), and may be 2 or less (e.g., 1.5 or less), preferably 1 or less (e.g., 0.8 or less), more preferably 0.5 or less (e.g., 0.3 or less), and particularly 0.2 or less (e.g., 0.18 or less, 0.15 or less, 0.12 or less, or 0.1 or less), and may be 0.0001 or more (e.g., 0.0005 or more, 0.001 or more, 0.005 or more, 0.01 or more, 0.02 or more, 0.03 or more, 0.04 or more, 0.05 or more, 0.06 or more, 0.07 or more, or 0.08 or more).

When the first purified product contains abn-CBD and the 2:1 adduct, the ratio of the proportion (e.g., the proportion on an HPLC area basis) of abn-CBD and the 2:1 adduct relative to the proportion of CBD [i.e., (the sum of the proportions of abn-CBD and the 2:1 adduct)/the proportion of CBD] in the first purified product may be selected from a range of, for example, about 10 or less (e.g., 8 or less), and may be 5 or less (e.g., 4 or less), preferably 3 or less (e.g., 2 or less), more preferably 1 or less (e.g., 0.8 or less), and particularly 0.5 or less (e.g., 0.4 or less, 0.3 or less, 0.2 or less, or 0.15 or less), and may be 0.0001 or more (e.g., 0.0005 or more, 0.001 or more, 0.01 or more, 0.05 or more, 0.07 or more, 0.08 or more, 0.1 or more, or 0.12 or more).

When the first purified product contains Δ9-THC, the ratio of the proportion (e.g., the proportion on an HPLC area basis) of Δ9-THC relative to the proportion of CBD (i.e., the proportion of Δ9-THC/the proportion of CBD) in the first purified product may be selected from a range of, for example, about 1 or less (e.g., 0.8 or less), and may be 0.5 or less (e.g., 0.4 or less), preferably 0.3 or less (e.g., 0.28 or less), more preferably 0.25 or less (e.g., 0.22 or less), and particularly 0.2 or less (e.g., 0.18 or less, 0.15 or less, 0.12 or less, 0.1 or less, 0.09 or less, 0.08 or less, 0.07 or less, 0.06 or less, 0.055 or less, 0.05 or less, 0.045 or less, or 0.04 or less), and may be 0.0001 or more (e.g., 0.0005 or more, 0.001 or more, 0.003 or more, 0.005 or more, 0.007 or more, 0.008 or more, 0.01 or more, 0.012 or more, 0.015 or more, 0.018 or more, 0.02 or more, 0.022 or more, 0.025 or more, 0.028 or more, or 0.03 or more).

When the first purified product contains Δ8-THC, the ratio of the proportion (e.g., the proportion on an HPLC area basis) of Δ8-THC relative to the proportion of CBD (i.e., the proportion of Δ8-THC/the proportion of CBD) in the first purified product may be selected from a range of, for example, about 0.1 or less (e.g., 0.08 or less), and may be 0.05 or less (e.g., 0.04 or less), preferably 0.03 or less (e.g., 0.028 or less), more preferably 0.025 or less (e.g., 0.022 or less), and particularly 0.02 or less (e.g., 0.018 or less, 0.015 or less, 0.012 or less, or 0.01 or less), and may be 0.00001 or more (e.g., 0.00005 or more, 0.0001 or more, 0.0003 or more, 0.0005 or more, 0.0007 or more, 0.0008 or more, 0.001 or more, 0.0012 or more, 0.0015 or more, 0.0018 or more, 0.002 or more, 0.0022 or more, 0.0025 or more, 0.0028 or more, 0.003 or more, 0.005 or more, or 0.007 or more).

When the first purified product contains Δ9-THC and Δ8-THC, the ratio of the proportion (e.g., the proportion on an HPLC area basis) of Δ9-THC and Δ8-THC relative to the proportion of CBD [i.e., (the sum of the proportions of Δ9-THC and Δ8-THC)/the proportion of CBD] in the first purified product may be selected from a range of, for example, about 1 or less (e.g., 0.8 or less), and may be 0.5 or less (e.g., 0.4 or less), preferably 0.3 or less (e.g., 0.28 or less), more preferably 0.25 or less (e.g., 0.22 or less), and particularly 0.2 or less (e.g., 0.18 or less, 0.15 or less, 0.12 or less, 0.1 or less, 0.09 or less, 0.08 or less, 0.07 or less, 0.06 or less, 0.055 or less, 0.05 or less, 0.045 or less, or 0.04 or less), and may be 0.0001 or more (e.g., 0.0005 or more, 0.001 or more, 0.003 or more, 0.005 or more, 0.007 or more, 0.008 or more, 0.01 or more, 0.012 or more, 0.015 or more, 0.018 or more, 0.02 or more, 0.022 or more, 0.025 or more, 0.028 or more, 0.03 or more, 0.032 or more, or 0.035 or more).

In the first purified product, the ratio of the proportion (e.g., the proportion on an HPLC area basis) of olivetol, abn-CBD, the 2:1 adduct, Δ9-THC, and Δ8-THC relative to the proportion of CBD [i.e., (the sum of the proportions of olivetol, abn-CBD, the 2:1 adduct, Δ9-THC, and Δ8-THC)/the proportion of CBD] may be selected from a range of, for example, about 20 or less (e.g., 10 or less), and may be 5 or less (e.g., 4 or less), preferably 3 or less (e.g., 2 or less), more preferably 1.5 or less (e.g., 1.2 or less), and particularly 1 or less (e.g., less than 1, 0.95 or less, 0.9 or less, 0.8 or less, 0.7 or less, 0.6 or less, 0.55 or less, 0.5 or less, 0.45 or less, 0.4 or less, 0.35 or less, 0.3 or less, or 0.25 or less), and may be 0.0001 or more (e.g., 0.0005 or more, 0.001 or more, 0.005 or more, 0.01 or more, 0.05 or more, 0.08 or more, 0.1 or more, 0.12 or more, 0.15 or more, 0.18 or more, or 0.2 or more).

When N1 denotes the ratio of the proportion (e.g., the proportion on an HPLC area basis) of olivetol, abn-CBD, the 2:1 adduct, Δ9-THC, and Δ8-THC relative to the proportion of CBD [i.e., (the sum of the proportions of olivetol, abn-CBD, the 2:1 adduct, Δ9-THC, and Δ8-THC)/the proportion of CBD] in the mixture, and N2 denotes the ratio of the proportion (e.g., the proportion on an HPLC area basis) of olivetol, abn-CBD, the 2:1 adduct, Δ9-THC, and Δ8-THC relative to the proportion of CBD [i.e., (the sum of the proportions of olivetol, abn-CBD, the 2:1 adduct, Δ9-THC, and Δ8-THC)/the proportion of CBD] in the first purified product, the value of "N1 - N2" may be greater than 0, and may be selected from a range of about 0.01 or more (e.g., 0.02 or more), and may be 0.03 or more (e.g., 0.05 or more), preferably 0.07 or more (e.g., 0.08 or more), more preferably 0.1 or more (e.g., 0.12 or more, 0.15 or more, 0.18 or more, 0.2 or more, 0.22 or more, 0.25 or more, 0.28 or more, or 0.3 or more).

In the first purified product, the ratio of the proportion (e.g., the proportion on an HPLC area basis) of abn-CBD, the 2:1 adduct, Δ9-THC, and Δ8-THC relative to the proportion of CBD [i.e., (the sum of the proportions of abn-CBD, the 2:1 adduct, Δ9-THC, and Δ8-THC)/the proportion of CBD] may be selected from a range of, for example, about 15 or less (e.g., 10 or less), and may be 5 or less (e.g., 4 or less), preferably 3 or less (e.g., 2 or less), more preferably 1.5 or less (e.g., 1.2 or less), and particularly 1 or less (e.g., less than 1, 0.95 or less, 0.9 or less, 0.8 or less, 0.7 or less, 0.6 or less, 0.55 or less, 0.5 or less, 0.45 or less, 0.4 or less, 0.35 or less, 0.3 or less, or 0.25 or less), and may be 0.0001 or more (e.g., 0.0005 or more, 0.001 or more, 0.005 or more, 0.01 or more, 0.05 or more, 0.08 or more, 0.1 or more, 0.12 or more, 0.15 or more, 0.18 or more, or 0.2 or more).

When N1 denotes the ratio of the proportion (e.g., the proportion on an HPLC area basis) of abn-CBD, the 2:1 adduct, Δ9-THC, and Δ8-THC relative to the proportion of CBD [i.e., (the sum of the proportions of abn-CBD, the 2:1 adduct, Δ9-THC, and Δ8-THC)/the proportion of CBD] in the mixture, and N2 denotes the ratio of the proportion (e.g., the proportion on an HPLC area basis) of abn-CBD, the 2:1 adduct, Δ9-THC, and Δ8-THC relative to the proportion of CBD [i.e., (the sum of the proportions of abn-CBD, the 2:1 adduct, Δ9-THC, and Δ8-THC)/the proportion of CBD] in the first purified product, the value of "N1 - N2" may be greater than 0, and may be selected from a range of about 0.01 or more (e.g., 0.02 or more), and may be 0.03 or more (e.g., 0.05 or more), preferably 0.07 or more (e.g., 0.08 or more), more preferably 0.1 or more (e.g., 0.12 or more, 0.15 or more, 0.18 or more, 0.2 or more, 0.22 or more, 0.25 or more, 0.28 or more, 0.3 or more, 0.32 or more, 0.35 or more, 0.38 or more, 0.4 or more, 0.42 or more, 0.45 or more, 0.48 or more, or 0.5 or more).

### Production of CBD from Solvate

CBD can be produced (manufactured, regenerated) from a solvate of CBD [e.g., a solvate obtained via CBD (crude CBD) derived from natural sources, a second purified product, crystals or crystallization products (obtained via CBD (crude CBD) derived from natural sources and/or obtained via a second purification step (e.g., a step of obtaining a second purified product via a first purified product))].

The method of producing CBD is not particularly limited as long as CBD can be produced by releasing (desolvating) the solvent (e.g., DMA) (the solvent associated with the solvate of CBD) from the solvate of CBD, and may be appropriately selected depending on factors such as the properties of the solvate of CBD.

Specific examples of the method include desolvation by heating the solvate of CBD, desolvation by dissolving the solvate of CBD in a solvent, and the like.

Typically, CBD may be produced by bringing a solvate of CBD (e.g., a DMA solvate of CBD) into contact with water (dissolving or dispersing it in water) accompanied by desolvation.

Conventional methods may be used for the separation (recovery) of the produced CBD, and in particular extraction may be used.

For example, by allowing a water-immiscible solvent (a solvent that phase-separates from water or forms a separate liquid phase from water, a hydrophobic solvent) that is capable of dissolving CBD (good solvent for CBD, extraction solvent, e.g., aliphatic hydrocarbons such as heptane) to coexist therewith, (by using such a water-immiscible solvent as an extraction solvent, a hydrophobic solvent capable of dissolving CBD), CBD (formed) can be transferred (separated, extracted) into the hydrophobic solvent layer (hydrophobic solvent layer, liquid-liquid phase-separated layer).

Examples of such hydrophobic solvents include the above exemplified solvents such as hydrocarbons.

In particular, such hydrophobic solvents may contain at least an aliphatic hydrocarbon {e.g., aliphatic hydrocarbons [e.g., aliphatic hydrocarbons (e.g., chain-structured or cyclic aliphatic hydrocarbons, saturated or unsaturated hydrocarbons, chain-structured or cyclic saturated aliphatic hydrocarbons, etc.) having 5 or more carbon atoms (e.g., 5 to 18 carbon atoms, 6 to 12 carbon atoms, 6 to 10 carbon atoms, etc.), etc.] such as pentane, hexane, isohexane, heptane, 2-methylhexane, 2,2-dimethylpentane, octane, isooctane, nonane, decane, cyclopentane, cyclohexane, cycloheptane, and methylcyclohexane}.

According to such extraction, even if the solvate of CBD contains other components (e.g., by-products), (in trace amounts thereof), further purification may be achieved (CBD having improved purity can be obtained).

For example, olivetol and by-products such as abn-CBD are typically present in only trace amounts in many cases, even when contained in the solvate. By further separating and removing such trace amounts of olivetol and by-products from CBD, it is possible to obtain CBD with even higher purity.

CBD can then be obtained from the liquid-liquid phase-separated layer (e.g., the hydrophobic solvent layer).

The liquid-liquid phase-separated layer (e.g., the hydrophobic solvent layer) may be further washed with a solvent (washing solvent) such as water.

The washing may be repeated once or two or more times.

The amount (amount used, total amount) of the washing solvent relative to 1 part by volume of the liquid-liquid phase-separated layer (e.g., the hydrophobic solvent layer) may be, for example, 0.1 or more (e.g., 0.5 or more, 1 or more, 2 or more, 3 or more, or 5 or more) and may be 30 or less (e.g., 20 or less, 15 or less, 10 or less, or 8 or less).

When the washing is carried out two or more times, the amount (amount used, total amount) of the washing solvent per wash relative to 1 part by volume of the liquid-liquid phase-separated layer (e.g., the hydrophobic solvent layer) may be, for example, 0.01 or more (e.g., 0.05 or more, 0.1 or more, 0.2 or more, 0.3 or more, or 0.5 or more) and may be 30 or less (e.g., 20 or less, 10 or less, 5 or less, 3 or less, 2 or less, or 1.5 or less).

CBD may be obtained by removing the solvent component from the liquid-liquid phase-separated layer (e.g., the hydrophobic solvent layer), and may also be obtained by causing crystal formation of CBD (by crystallizing CBD). Such crystallization may enable further purification (CBD may be purified to a higher degree of purity) in the same manner as described above.

The crystal formation of CBD is not particularly limited and may be carried out using conventional methods; for example, crystallization (recrystallization) may be employed.

The solvent used for the crystallization (recrystallization) is not particularly limited, and the above-described solvents may be used. Examples of the solvents include hydrocarbons [e.g., aliphatic hydrocarbons (e.g., pentane, hexane, isohexane, heptane, 2-methylhexane, 2,2-dimethylpentane, octane, isooctane, nonane, decane, cyclopentane, cyclohexane, cycloheptane, methylcyclohexane, etc.), aromatic hydrocarbons (e.g., benzene, toluene, xylene, ethylbenzene, etc.)], ketones (e.g., acetone, methyl ethyl ketone, methyl isobutyl ketone, etc.), ethers [e.g., chain-structured ethers (e.g., diethyl ether, methyl tert-butyl ether, etc.), cyclic ethers (e.g., tetrahydrofuran, dioxane, etc.)] and the like.

Among these, there may particularly be used at least aliphatic hydrocarbons {e.g., aliphatic hydrocarbons [e.g., aliphatic hydrocarbons (e.g., chain-structured or cyclic aliphatic hydrocarbons, saturated or unsaturated hydrocarbons, chain-structured or cyclic saturated aliphatic hydrocarbons, etc.) having 5 or more carbon atoms (e.g., 5 to 18 carbon atoms, 6 to 12 carbon atoms, 6 to 10 carbon atoms, etc.] such as pentane, hexane, isohexane, heptane, 2-methylhexane, 2,2-dimethylpentane, octane, isooctane, nonane, decane, cyclopentane, cyclohexane, cycloheptane, and methylcyclohexane, and the like}.

In crystallization (recrystallization), the proportion (amount used) of the solvent may be selected depending on factors such as the crystallization conditions; for example, the proportion (concentration) may be such that the solid content concentration is 1 mass% or more (e.g., 5 mass% or more, 10 mass% or more, 20 mass% or more, or 30 mass% or more) and 90 mass% or less (e.g., 80 mass% or less, 70 mass% or less, 60 mass% or less, or 50 mass% or less).

The reaction (crystallization) may be carried out in the presence of seed crystals.

The crystallization (recrystallization) may be carried out at room temperature (or ambient temperature), under heating, or under cooling.

The crystallization (recrystallization) temperature may be, specifically, for example, -100°C or more (e.g., -80°C or more, -60°C or more, -40°C or more, -20°C or more, -10°C or more, 0°C or more, 10°C or more, 20°C or more, 30°C or more, 35°C or more, or 40°C or more) and 150°C or less (e.g., 140°C or less, 130°C or less, 120°C or less, 110°C or less, 100°C or less, 90°C or less, 80°C or less, 70°C or less, 60°C or less, or 50°C or less).

In particular, the crystallization (recrystallization) temperature may be set to a relatively low or not excessively high temperature (e.g., 40°C or less, 35°C or less, 30°C or less, 25°C or less, 20°C or less, 15°C or less, 10°C or less, or 5°C or less). The crystallization (recrystallization) temperature may also be decreased stepwise.

The crystallization (recrystallization) time may be appropriately selected depending on factors such as the degree of progress of crystallization (recrystallization) and various crystallization (recrystallization) conditions, and is not particularly limited. For example, the crystallization (recrystallization) time may be 30 seconds or more (e.g., 1 minute or more, 2 minutes or more, 5 minutes or more, 10 minutes or more, 15 minutes or more, 20 minutes or more, 25 minutes or more, 30 minutes or more, 40 minutes or more, 60 minutes or more, 1.5 hours or more, or 2 hours or more), and may be 48 hours or less (e.g., 36 hours or less, 24 hours or less, 18 hours or less, 12 hours or less, or 6 hours or less).

The crystallization (recrystallization) may be carried out under stirring or may be carried out while standing.

The crystallization (recrystallization) may also be carried out under an inert atmosphere (e.g., nitrogen or argon) and the like.

The vessel used for the crystallization (recrystallization) is not particularly limited, and conventional vessels may be used.

Through the above reaction (crystallization), CBD is produced (manufactured, recovered, regenerated).

It should be noted that CBD may be recovered substantially completely. For example, when A1 denotes the mass of CBD in the solvate of CBD and A2 denotes the mass of CBD (regenerated CBD), the value of A2/A1 may be selected from a range of, for example, about 0.7 or more, and may be 0.8 or more (e.g., 0.85 or more, 0.88 or more, 0.9 or more, 0.92 or more, 0.93 or more, 0.94 or more, 0.95 or more, 0.96 or more, 0.97 or more, 0.98 or more, 0.99 or more, or substantially 1).

Further, CBD may or may not contain components other than CBD (e.g., olivetol, by-products, or other (additional) cannabinoids). Even when components other than CBD are present, they are often present only in extremely small amounts (i.e., the CBD is highly purified).

For example, in CBD (regenerated CBD), the proportion (e.g., the proportion on an HPLC area (peak area) basis) of CBD relative to the total amount of CBD and other cannabinoids [or CBG, CBN, CBC, CBDV, and THCs (e.g., Δ9-THC, Δ8-THC)] [or CBG, CBN, and THCs (e.g., Δ9-THC, Δ8-THC)] may be selected from a range of, for example, 88% or more (e.g., 90% or more), and may be 92% or more (e.g., 93% or more), preferably 95% or more (e.g., 96% or more), more preferably 97% or more (e.g., 97.5% or more), and particularly 98% or more, 98.5% or more, 99% or more, 99.2% or more, 99.5% or more, 99.9% or more, 99.95% or more, or substantially 100%.

Further, in CBD (regenerated CBD), the proportion (e.g., the proportion on an HPLC area (peak area) basis) of CBD relative to the total amount of CBD, olivetol, abn-CBD, the 2:1 adduct, Δ9-THC, and Δ8-THC may be selected from a range of, for example, 88% or more (e.g., 90% or more), and may be 92% or more (e.g., 93% or more), preferably 95% or more (e.g., 96% or more), more preferably 97% or more (e.g., 97.5% or more), and particularly 98% or more, 98.5% or more, 99% or more, 99.2% or more, 99.5% or more, 99.9% or more, 99.95% or more, or substantially 100%.

CBD (regenerated CBD) may or may not contain olivetol. Even when olivetol is present, olivetol is often present only in a small amount. For example, in CBD (regenerated CBD), the proportion (e.g., the proportion on an HPLC area basis) of olivetol relative to the total amount of CBD, olivetol, abn-CBD, the 2:1 adduct, Δ9-THC, and Δ8-THC may be, for example, 8% or less (e.g., 7% or less, 6% or less, 5% or less, 4% or less, 3% or less, 2% or less, 1.5% or less, 1.2% or less, 1% or less, 0.9% or less, 0.8% or less, 0.7% or less, 0.6% or less, 0.5% or less, 0.4% or less, 0.3% or less, 0.2% or less, 0.1% or less, 0.01% or less, or 0.001% or less).

CBD (regenerated CBD) may or may not contain abn-CBD. Even when abn-CBD is present, abn-CBD is often present only in a small amount. For example, in CBD (regenerated CBD), the proportion (e.g., the proportion on an HPLC area basis) of abn-CBD relative to the total amount of CBD, olivetol, abn-CBD, the 2:1 adduct, Δ9-THC, and Δ8-THC may be, for example, 8% or less (e.g., 7% or less, 6% or less, 5% or less, 4% or less, 3% or less, 2% or less, 1.5% or less, 1.2% or less, 1% or less, 0.9% or less, 0.8% or less, 0.7% or less, 0.6% or less, 0.5% or less, 0.4% or less, 0.3% or less, 0.2% or less, 0.1% or less, 0.01% or less, or 0.001% or less).

CBD (regenerated CBD) may or may not contain the 2:1 adduct. Even when the 2:1 adduct is present, the 2:1 adduct is often present only in a small amount. For example, in CBD (regenerated CBD), the proportion (e.g., the proportion on an HPLC area basis) of the 2:1 adduct relative to the total amount of CBD, olivetol, abn-CBD, the 2:1 adduct, Δ9-THC, and Δ8-THC may be, for example, 3% or less (e.g., 2.5% or less, 2% or less, 1.5% or less, 1.2% or less, 1% or less, 0.9% or less, 0.8% or less, 0.7% or less, 0.6% or less, 0.5% or less, 0.4% or less, 0.3% or less, 0.2% or less, 0.1% or less, 0.05% or less, 0.01% or less, 0.001% or less, or 0.0001% or less).

CBD (regenerated CBD) may or may not contain other cannabinoids. Even when other cannabinoids are present, they are often present only in a small amount. For example, in CBD (regenerated CBD), the proportion (e.g., the proportion on an HPLC area basis) of other cannabinoids [or CBG, CBN, CBC, CBDV, and THCs (e.g., Δ9-THC, Δ8-THC)] [or CBG, CBN, and THCs (e.g., Δ9-THC, Δ8-THC)] relative to the total amount of CBD and other cannabinoids [or CBG, CBN, CBC, CBDV, and THCs (e.g., Δ9-THC, Δ8-THC)] [or CBG, CBN, and THCs (e.g., Δ9-THC, Δ8-THC)] may be, for example, 5% or less, 4% or less, 3% or less, 2% or less, 1.5% or less, 1.2% or less, 1% or less, 0.9% or less, 0.8% or less, 0.7% or less, 0.6% or less, 0.5% or less, 0.4% or less, 0.3% or less, 0.2% or less, 0.1% or less, 0.05% or less, 0.01% or less, 0.001% or less, or 0.0001% or less.

CBD (regenerated CBD) may or may not contain Δ9-THC. Even when Δ9-THC is present, Δ9-THC is often present only in a small amount. For example, in CBD (regenerated CBD), the proportion (e.g., the proportion on an HPLC area basis) of Δ9-THC relative to the total amount of CBD, olivetol, abn-CBD, the 2:1 adduct, Δ9-THC, and Δ8-THC may be, for example, 1% or less (e.g., 0.9% or less, 0.8% or less, 0.7% or less, 0.6% or less, 0.5% or less, 0.4% or less, 0.3% or less, 0.2% or less, 0.1% or less, 0.05% or less, 0.01% or less, 0.001% or less, or 0.0001% or less).

CBD (regenerated CBD) may or may not contain Δ8-THC. Even when Δ8-THC is present, Δ8-THC is often present only in a small amount. For example, in CBD (regenerated CBD), the proportion (e.g., the proportion on an HPLC area basis) of Δ8-THC relative to the total amount of CBD, olivetol, abn-CBD, the 2:1 adduct, Δ9-THC, and Δ8-THC may be, for example, 1% or less (e.g., 0.9% or less, 0.8% or less, 0.7% or less, 0.6% or less, 0.5% or less, 0.4% or less, 0.3% or less, 0.2% or less, 0.1% or less, 0.05% or less, 0.01% or less, 0.001% or less, or 0.0001% or less).

CBD (regenerated CBD) may or may not contain Δ9-THC and Δ8-THC. Even when Δ9-THC and Δ8-THC are present, they are often present only in small amounts. For example, in CBD (regenerated CBD), the proportion (e.g., the proportion on an HPLC area basis) of Δ9-THC and Δ8-THC relative to the total amount of CBD, olivetol, abn-CBD, the 2:1 adduct, Δ9-THC, and Δ8-THC may be, for example, 1% or less (e.g., 0.9% or less, 0.8% or less, 0.7% or less, 0.6% or less, 0.5% or less, 0.4% or less, 0.3% or less, 0.2% or less, 0.1% or less, 0.05% or less, 0.01% or less, 0.001% or less, or 0.0001% or less).

When CBD (regenerated CBD) contains olivetol, the ratio of the proportion (e.g., the proportion on an HPLC area basis) of olivetol relative to the proportion of CBD (i.e., the proportion of olivetol/the proportion of CBD) in CBD (regenerated CBD) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, or 0.0001 or less).

When CBD (regenerated CBD) contains abn-CBD, the ratio of the proportion (e.g., the proportion on an HPLC area basis) of abn-CBD relative to the proportion of CBD (i.e., the proportion of abn-CBD/the proportion of CBD) in CBD (regenerated CBD) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, or 0.0001 or less).

When CBD (regenerated CBD) contains the 2:1 adduct, the ratio of the proportion (e.g., the proportion on an HPLC area basis) of the 2:1 adduct relative to the proportion of CBD (i.e., the proportion of the 2:1 adduct/the proportion of CBD) in CBD (regenerated CBD) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, 0.0005 or less, or 0.0001 or less).

When CBD (regenerated CBD) contains Δ9-THC, the ratio of the proportion (e.g., the proportion on an HPLC area basis) of Δ9-THC relative to the proportion of CBD (i.e., the proportion of Δ9-THC/the proportion of CBD) in CBD (regenerated CBD) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, 0.0005 or less, or 0.0001 or less).

When CBD (regenerated CBD) contains Δ8-THC, the ratio of the proportion (e.g., the proportion on an HPLC area basis) of Δ8-THC relative to the proportion of CBD (i.e., the proportion of Δ8-THC/the proportion of CBD) in CBD (regenerated CBD) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, 0.0005 or less, or 0.0001 or less).

When CBD (regenerated CBD) contains Δ9-THC and Δ8-THC, the ratio of the proportion (e.g., the proportion on an HPLC area basis) of Δ9-THC and Δ8-THC relative to the proportion of CBD [i.e., (the sum of the proportions of Δ9-THC and Δ8-THC)/the proportion of CBD] in CBD (regenerated CBD) may be selected from a range of, for example, about 0.1 or less (e.g., 0.05 or less), and may be 0.03 or less (e.g., 0.025 or less), preferably 0.02 or less (e.g., 0.015 or less), more preferably 0.01 or less (e.g., 0.008 or less), and particularly 0.005 or less (e.g., 0.004 or less, 0.003 or less, 0.002 or less, 0.001 or less, 0.0005 or less, or 0.0001 or less).

When CBD (regenerated CBD) contains at least one selected from the group consisting of olivetol, abn-CBD, the 2:1 adduct, Δ9-THC, and Δ8-THC, the ratio of the proportion (e.g., the proportion on an HPLC area basis) of olivetol, abn-CBD, the 2:1 adduct, Δ9-THC, and Δ8-THC relative to the proportion of CBD [i.e., (the sum of the proportions of olivetol, abn-CBD, the 2:1 adduct, Δ9-THC, and Δ8-THC)/the proportion of CBD] in CBD (regenerated CBD) may be selected from a range of, for example, about 0.5 or less (e.g., 0.3 or less), and may be 0.1 or less (e.g., 0.08 or less), preferably 0.05 or less (e.g., 0.03 or less), more preferably 0.02 or less (e.g., 0.015 or less), and particularly 0.01 or less (e.g., 0.008 or less, 0.006 or less, 0.004 or less, 0.003 or less, 0.002 or less, 0.001 or less, 0.0005 or less, or 0.0001 or less).

CBD (regenerated CBD) is generally free from solvent [residual solvent, such as a solvent derived from a solvate (a solvent constituting the solvate, such as dimethylacetamide) and a solvent used during regeneration (e.g., an aliphatic hydrocarbon such as heptane)]; (CBD (regenerated CBD) contains such solvent below the detection limit). Alternatively, even when CBD (regenerated CBD) contains a solvent, the amount thereof may be only trace.

For example, when CBD (regenerated CBD) contains a solvent (solvent component), the proportion of the solvent relative to the total CBD (CBD containing residual solvent) may be, for example, 5 mass% or less (e.g., 4 mass% or less, 3.5 mass% or less, 3 mass% or less, 2.5 mass% or less, 2 mass% or less, 1.5 mass% or less, 1.2 mass% or less, 1 mass% or less, 0.8 mass% or less, 0.7 mass% or less, 0.6 mass% or less, 0.5 mass% or less, 0.3 mass% or less, 0.2 mass% or less, or 0.1 mass% or less).

More specifically, the proportion (upper limit) of the solvent derived from the solvate (the solvent constituting the solvate, such as dimethylacetamide) relative to the total CBD may be 5 mass% or less, 4.5 mass% or less, 4 mass% or less, 3.5 mass% or less, 3 mass% or less, 2.5 mass% or less, 2 mass% or less, 1.5 mass% or less, 1.2 mass% or less, 1 mass% or less, 0.8 mass% or less, 0.5 mass% or less, 0.3 mass% or less, 0.2 mass% or less, or 0.1 mass% or less.

In addition, the proportion (upper limit) of hydrocarbons (e.g., aliphatic hydrocarbons such as heptane) relative to the total CBD may be 3 mass% or less, 2.5 mass% or less, 2 mass% or less, 1.5 mass% or less, 1.2 mass% or less, 1 mass% or less, 0.8 mass% or less, 0.5 mass% or less, 0.3 mass% or less, 0.2 mass% or less, 0.1 mass% or less, 0.08 mass% or less, or 0.05 mass% or less.

It should be noted that when CBD (regenerated CBD) contains a solvent (solvent component), the proportion of the solvent relative to the total CBD (CBD containing residual solvent) may be at or above the detection limit, and may be, for example, 0.001 mass% or more, 0.005 mass% or more, 0.01 mass% or more, 0.02 mass% or more, 0.03 mass% or more, 0.05 mass% or more, 0.06 mass% or more, 0.07 mass% or more, 0.08 mass% or more, 0.09 mass% or more, or 0.1 mass% or more.

More specifically, the proportion (lower limit) of the solvent derived from the solvate (a solvent constituting the solvate, such as dimethylacetamide) relative to the total CBD may be 0.001 mass% or more, 0.005 mass% or more, 0.01 mass% or more, 0.05 mass% or more, 0.1 mass% or more, 0.5 mass% or more, or 1 mass% or more.

In addition, the proportion (lower limit) of hydrocarbons (e.g., aliphatic hydrocarbons such as heptane) relative to the total CBD may be 0.0001 mass% or more, 0.0005 mass% or more, 0.0001 mass% or more, 0.001 mass% or more, 0.005 mass% or more, 0.01 mass% or more, 0.05 mass% or more, 0.1 mass% or more, or 0.5 mass% or more.

The proportion of the solvent may be measured, for example, by gas chromatography or the like.

CBD (regenerated CBD) may particularly be crystalline (in crystalline form).

### EXAMPLES

The present invention will be described in detail by way of the following Examples, but the present invention is not limited thereto.

The various analyses and measurements (calculations) were performed as follows.

### HPLC

Measurements were performed using the following apparatus and conditions.
Instrument: LC-2010HT manufactured by Shimadzu Corporation
Detection wavelength: 210 nm
Column: Xselect CSH C18, 4.6 mm × 150 mm (particle size: 3.5 µm) manufactured by Waters Corporation
Column temperature: 35°C
Mobile phase A: 0.1% formic acid aqueous solution
Mobile phase B: 0.1% formic acid acetonitrile solution
Flow rate: 1.0 mL/min.
Gradient: shown in Table 1

**Table 1**

| Time (min.) | Mobile Phase A (%) | Mobile Phase B (%) |
|---|---|---|
| 0.0 | 60 | 40 |
| 5.0 | 50 | 50 |
| 5.1 | 15 | 85 |
| 11.0 | 15 | 85 |
| 11.1 | 5 | 95 |
| 18.0 | 5 | 95 |
| 18.1 | 60 | 40 |
| 23.0 | 60 | 40 |

The "%" values represent percentages based on the total of peak areas from 0.0 to 18.0 minutes being taken as 100%.

### Powder X-ray (2θ)

Measurements were performed using the following apparatus and conditions.
Instrument: SmartLab manufactured by Rigaku Corporation
Measurement method: Reflection method
X-ray source: Cu tube
Wavelength used: Cu Kβ ray
Tube current: 200 mA
Tube voltage: 45 kV
Sample plate: Circular non-reflective sample holder
X-ray incident angle: 4° to 40°

### ¹H-NMR

Measured using Ascend^{™} 400 manufactured by Bruker

### Melting point

The melting point was determined based on the peak temperature of the endothermic event measured by DSC under the following apparatus and conditions.
Instrument: DSC 3+ manufactured by METTLER TOLEDO
Conditions: Approximately 1.2 mg of sample was weighed, placed in an aluminum pan, and measured at a temperature range of 60°C to 70°C at a heating rate of 0.5°C/min.

### Gas Chromatography

Measurements were performed under the following conditions.
Detector: FID
Column: DB-624 (30 m × 0.320 mm, 1.80 µm)
Column temperature: 35°C (0 min) → 3°C/min → 70°C (0 min) → 9°C/min → 130°C (5 min) → 15°C/min → 230°C (0 min)
Injection port temperature: 200°C
Detector temperature: 230°C
Carrier gas: N₂
Flow control mode: Constant flow
Split ratio: 40:1
Injection volume: 1.0 µL
Area measurement range: 30 minutes
Sample diluent solvent: N-methylpyrrolidone

It should be noted that the retention times of residual solvents were as follows: MTBE (8.0 min), heptane (13.0 min), toluene (16.5 min), DMA (21.6 min), and the like.

### Example 1

Cannabidiol (CBD) was dissolved in DMA [1.6 V (1.6 times by volume of DMA relative to CBD)], and then 0.3 V of water was added. The obtained solution was rapidly cooled to 5°C, and after confirming precipitation of a solid (crystals), the slurry was filtered to obtain the solid (crystals).

Separately, heptane (10 V), DMA (3 eq.), and toluene (0.3 V) were added to cannabidiol and the mixture was rapidly cooled to 5°C to obtain a solid (crystals).

Analysis of the obtained solids (crystals) confirmed that in each case, the compound was a solvate in which 2 mol of DMA were added (solvated) per (relative to) 1 mol of cannabidiol (CBD-2DMA).

The analysis results are as follows.

### • Powder X-ray (2θ):

The characteristic peaks (C) as described above (i.e., approximately 20 peaks listed below) were confirmed.

8.3 ± 0.2°, 8.5 ± 0.2°, 10.6 ± 0.2°, 11.8 ± 0.2°, 13.4 ± 0.2°, 15.4 ± 0.2°, 15.8 ± 0.2°, 16.4 ± 0.2°, 17.0 ± 0.2°, 18.9 ± 0.2°, 19.9 ± 0.2°, 21.4 ± 0.2°, 22.7 ± 0.2°, 22.9 ± 0.2°, 23.2 ± 0.2°, 23.7 ± 0.2°, 24.6 ± 0.2°, 26.5 ± 0.2°, 27.1 ± 0.2°, 27.7 ± 0.2°

### •¹H-NMR

δ ppm: 0.88 (t, J = 7.2 Hz, 3H), 1.22-1.36 (m, 2H), 1.52-1.60 (m, 2H), 1.64-1.67 (m, 3H), 1.75-1.85 (m, 2H), 1.78-1.80 (m, 3H), 2.07-2.13 (m, 2H), 2.08 (s, 6H), 2.18-2.28 (m, 3H), 2.37-2.46 (m, 3H), 2.94 (s, 6H), 3.01 (s, 6H), 3.82-3.87 (m, 1H), 4.56 (s, 1H), 4.65-4.67 (m, 1H), 5.56-5.58 (m, 1H), 6.13-6.30 (m, 1H)

### • Melting point: 52-55°C [by the above apparatus and conditions (DSC)]

It should be noted that the obtained solid (crystals, CBD-2DMA) had less solubility in various solvents such as heptane compared to CBD.

### Synthesis Example 1

Olivetol (21.63 parts by mass) containing 9.3 mass% water [i.e., 19.62 parts by mass of olivetol (1.09 eq. relative to menthadienol) and 2.01 parts by mass of water] was azeotropically dehydrated three times with 171.52 parts by mass of toluene [13 V (13 times by volume relative to menthadienol)], after which the amount of toluene was adjusted to 20 V in the reaction vessel.

Zinc iodide (ZnI₂) (3.19 parts by mass, 0.1 eq. relative to menthadienol) was added thereto, and the temperature was adjusted to 110°C.

A solution of 15.22 parts by mass of menthadienol in 171.54 parts by mass of toluene (13 V) was added dropwise over 30 minutes [i.e., the total volume of solvent (toluene) was 33 V]. After the dropwise addition, the mixture was allowed to react for 5 minutes, and then cooled to room temperature over 1 hour to obtain a reaction mixture (reaction solution).

It should be noted that the proportion of water in the reaction vessel (reaction system) was 0.04 mass% at the time of addition of menthadienol (at the start of the reaction, immediately before addition).

The composition of the reaction solution was confirmed by HPLC to be as follows: CBD 62.96%, olivetol 13.26%, abn-CBD 12.17%, Δ9-THC 2.20%, Δ8-THC 0.28%, and the 2:1 adduct 5.46%. The quantitative value was calculated to be 63.40%.

It should be noted that the quantitative value as used herein refers to the yield (calculated value) calculated based on the amount of CBD in the liquid as determined by HPLC using a standard substance (CBD standard); the same applies hereinafter.

### Synthesis Example 2

Tetrahydrofuran (20 V) and boron trifluoride diethyl ether (BF₃·OEt₂) (85.3 mg (1.8 eq. relative to menthadienol)) were added to a reaction vessel, and the temperature was adjusted to -10°C using a jacket.

To the reaction vessel were added (fed) olivetol (21.63 parts by mass) containing 9.3 mass% water [i.e., 19.62 parts by mass of olivetol (1.09 eq. relative to menthadienol) and 2.01 parts by mass of water] and a solution of 15.22 parts by mass of menthadienol in 171.54 parts by mass of toluene (13 V) [i.e., the total volume of solvent (toluene) was 33 V], and the mixture was allowed to react for 3.5 hours after addition (feeding), and then cooled to room temperature over 1 hour to obtain a reaction mixture (reaction solution).

The composition of the reaction solution was confirmed by HPLC to be as follows: CBD 13.97%, olivetol 31.365%, abn-CBD 45.02%; Δ9-THC and Δ8-THC were below the detection limit.

As is clear from the composition of components in each reaction solution, by selecting the synthesis method, the method of Synthesis Example 1 reduced the amount of unreacted components (olivetol) and by-products relative to Synthesis Example 2; however, such unreacted components (olivetol) and by-products were still present in the reaction mixture.

### Example 2

### Crude Product Preparation Step

In the crude product preparation step, the mass of menthadienol used in the reaction was taken as the basis for wt (mass) and V (volume).

The reaction solution obtained in Synthesis Example 1 was concentrated under reduced pressure to 2.9 wt, followed by addition of heptane (104.10 parts by mass, 10 V), and then the mixture was concentrated again under reduced pressure to replace the solvent with heptane.

Heptane (343.64 parts by mass, 33 V) and silica gel (45.66 parts by mass, 3 wt, particle size 40-50 µm, neutral, spherical) were added to the concentrated solution, and the resulting mixture was stirred at room temperature for 30 minutes.

The mixture was filtered to remove the silica gel and washed with 103.42 parts by mass (10 V) of a toluene-heptane solution (3 vol% toluene). The filtrate was concentrated under reduced pressure to 1.6 wt to obtain a crude product (mixture, first purified product).

The composition of the crude product was confirmed by HPLC to be as follows: CBD 79.91%, olivetol 0.41%, abn-CBD 4.63%, Δ9-THC 2.95%, Δ8-THC 0.72%, the 2:1 adduct 7.9%. The quantitative value was calculated as 55.7%.

### Purification Step

In the purification step, the quantitative value of cannabidiol in the crude product (mixture, first purified product) was taken as the basis for wt (mass), V (volume), and eq.

### (equivalents).

Heptane (107.85 parts by mass, 9 V) and methyl t-butyl ether (MTBE) (12.96 parts by mass, 1 V) were added to the obtained crude product. After the resulting solution was confirmed to be a single layer, the solution was cooled to 10°C.

DMA (4.58 parts by mass, 1 eq.) was added dropwise thereto, followed by addition of 0.0127 parts by mass (0.07 wt% based on the quantitative value of CBD in the crude composition) of crystals (seed crystals) derived from the CBD-2DMA crystals obtained in Example 1.

After confirming precipitation of crystals, DMA (9.70 parts by mass, 2 eq.) was added dropwise, and the mixture was cooled to 0-5°C. The mixture was aged for crystallization at 0-5°C for 2 hours, after which the crystals were filtered. The crystals were washed with a washing solvent (43.17 parts by mass, 3.5 V) [heptane: MTBE: DMA = 9:1:0.3 (volume ratio)], and dried under a nitrogen stream for 20 minutes.

The obtained crystals (solid) were confirmed to be CBD-2DMA (to contain CBD-2DMA) in the same manner as described above.

Heptane (71.90 parts by mass, 6 V) and tap water (105.12 parts by mass, 6 V) were added to the crystals (solid), and after stirring, complete dissolution of the solid was confirmed.

After liquid-liquid phase separation, the organic layer was washed three times with 105.12 parts by mass (6 V) of tap water, and the washed organic layer was dried with 35.04 parts by mass (2 wt) of sodium sulfate.

The sodium sulfate was filtered off, and the filtered solution was concentrated to 1.0 wt under reduced pressure.

The composition of the washed organic layer was confirmed by HPLC to be as follows: CBD 99.63%, olivetol 0.1%, abn-CBD 0.27%; Δ9-THC, Δ8-THC, and the 2:1 adduct were not detected. The quantitative value was calculated as 51.8%.

In the subsequent purification steps, the quantitative value of cannabidiol in the washed organic layer was taken as the basis for wt (mass) and V (volume).

Heptane (22.28 parts by mass, 2 V relative to the quantitative value of CBD) was added to the concentrated solution, followed by heating to 30°C. CBD seed crystals (0.1722 parts by mass, 1 wt% relative to the quantitative value of CBD) were then added, and after confirming precipitation of crystals, crystallization aging was carried out at 25°C for 1 hour.

The mixture was then cooled to 0-5°C over 20 minutes and, after 45 minutes of crystallization aging, was further cooled to -15°C over 15 minutes. After 2 hours of crystallization aging, the crystals (solid) were filtered and washed with 12.07 parts by mass (1.1 V) of heptane cooled to the same temperature. The crystals (solid) were dried by aeration for 25 minutes and then dried under reduced pressure at 40°C (jacket temperature) for 1 hour to obtain 14.92 parts by mass of solid (crystals).

The obtained solid (crystals) was confirmed to be CBD by ¹H-NMR analysis.

That is, it was found that the obtained solid (crystals, CBD-2DMA) released DMA upon contact with water (in water) to form CBD (regenerates CBD).

The composition of the solid (CBD crystals) was confirmed by HPLC to be as follows: CBD 100.00%; olivetol, abn-CBD, Δ9-THC, Δ8-THC, and the 2:1 adduct were not detected. The yield of CBD (yield assuming 100% reaction of menthadienol) was calculated as 46.9%

### (calculated by subtracting the mass of seed crystals).

The obtained solid (CBD crystals) contained a very small amount of residual (remaining) solvent (176 mass ppm of heptane, 251 mass ppm of DMA).

Accordingly, various additional or modified operations were carried out in the purification step, whereby the amount of residual solvents could be reduced. For example, when the operation of washing the above-mentioned organic layer three times with 105.12 parts by mass (6 V) of tap water was changed to an operation of washing the organic layer three times with 105.12 parts by mass (6 V) of tap water and four times with 122.64 parts by mass (7 V) of tap water, the proportion of DMA in the obtained solid (CBD) fell below the detection limit (DMA was not detected).

It should be noted that, although CBD-2DMA was used as seed crystals in the above method, it has been confirmed that crystals can be obtained (crystallization or precipitation occurs) even in the absence of seed crystals.

### Example 3

### Crude Product Preparation Step

In the crude product preparation step, the mass of menthadienol used in the reaction was taken as the basis for wt (mass) and V (volume).

A reaction solution was obtained by the same method as in Synthesis Example 1.

The composition of the reaction solution was confirmed by HPLC to be as follows: CBD 62.897%, olivetol 13.764%, abn-CBD 10.695%, Δ9-THC 3.067%, Δ8-THC 0.412%, and the 2:1 adduct 4.642%. The quantitative value was calculated as 64.77%.

This reaction solution was concentrated under reduced pressure to 2.49 wt, and heptane (6.6 V) was added followed by concentration under reduced pressure; this operation was repeated twice to replace the solvent with heptane.

Heptane (33 V) and silica gel (45.6 parts by mass, 3 wt, particle size 40-50 µm, neutral, spherical) were added to the concentrated solution, and the resulting mixture was stirred at room temperature for 1 hour.

The mixture was filtered to remove the silica gel and washed with 10 V of a toluene-heptane solution (3 vol% toluene). The filtrate was concentrated under reduced pressure to 1.7 wt to obtain a crude product.

The composition of the crude product was confirmed by HPLC to be as follows: CBD 76.47%, olivetol 0.28%, abn-CBD 3.7%, Δ9-THC 4.32%, Δ8-THC 1.25%, and the 2:1 adduct 11.65%. The quantitative value was calculated as 52.5%.

### Purification Step

In the purification step, the quantitative value of cannabidiol in the crude product (mixture, first purified product) was taken as the basis for wt (mass), V (volume), and eq.

### (equivalents).

Heptane (9 V) and methyl t-butyl ether (MTBE, 1 V) were added to the crude product. After the resulting solution was confirmed to be a single layer, the solution was cooled to 10°C.

DMA (1 eq.) was added dropwise thereto, followed by addition of crystals derived from CBD-2DMA crystals obtained in Example 1 as seed crystals.

After confirming precipitation of crystals, DMA (2 eq.) was added dropwise and the mixture was cooled to 0-5°C. The mixture was aged for crystallization at 0-5°C for 1 hour, after which the crystals were filtered. The crystals were washed with a washing solvent (1.8 V) [heptane: MTBE: DMA = 9:1:0.3 (volume ratio)], and then dried under a nitrogen stream for 30 minutes.

Heptane (5.7 V) and tap water (5.7 V) were added to the crystals (solid), and after stirring, complete dissolution of the solid was confirmed.

After liquid-liquid phase separation, the organic layer was washed three times with 5.7 V of tap water, and the washed organic layer was dried with sodium sulfate (7.05 parts by mass, 4.3 wt).

The sodium sulfate was filtered off, the solution was concentrated under reduced pressure to 1.0 wt, and then heptane (3.0 V) was added.

The composition of the washed organic layer was confirmed by HPLC to be as follows: CBD 99.06%, olivetol 0.03%, abn-CBD 0.91%; Δ9-THC, Δ8-THC, and the 2:1 adduct were not detected. The quantitative value was calculated as 49.41%.

In the subsequent purification steps, the quantitative value of cannabidiol in the washed organic layer was taken as the basis for wt (mass) and V (volume).

Heptane (3 V) was added to the concentrated solution, and after heating to 25°C, CBD seed crystals (0.0009 parts by mass, 0.06 wt%) were added; after confirming precipitation of crystals, crystallization aging was carried out at 25°C for 1 hour. The mixture was then cooled to -10°C and crystallization aging was carried out for 1 hour. The crystals (solid) were filtered and washed with heptane cooled to the same temperature. The crystals (solid) were dried by aeration for 15 minutes and then dried under reduced pressure at 40°C (jacket temperature) for 1 hour to obtain 1.25 parts by mass of solid (crystals).

The composition of the solid (CBD crystals) was confirmed by HPLC to be as follows: CBD 100.00%; olivetol, abn-CBD, Δ9-THC, Δ8-THC, and the 2:1 adduct were not detected. The yield of CBD (yield assuming 100% reaction of menthadienol) was calculated as 40.3% (calculated by subtracting the mass of seed crystals).

### Example 4

### Crude Product Preparation Step

In the crude product preparation step, the mass of menthadienol used in the reaction was taken as the basis for wt (mass) and V (volume).

A reaction solution was obtained by the same method as in Synthesis Example 1.

The composition of the reaction solution was confirmed by HPLC to be as follows: CBD 62.897%, olivetol 13.764%, abn-CBD 10.695%, Δ9-THC 3.067%, Δ8-THC 0.412%, the 2:1 adduct 4.642%. The quantitative value was calculated as 64.77%.

The concentrate of this reaction solution was used in the following purification step.

### Purification Step

In the purification step, the quantitative value of cannabidiol in the concentrate was taken as the basis for wt (mass), V (volume), and eq. (equivalents).

Heptane (4.5 V) and methyl t-butyl ether (MTBE) (0.5 V) were added to the concentrate of the reaction solution. After the resulting solution was confirmed to be a single layer, the solution was cooled to 0°C.

DMA (2 eq.) was added dropwise thereto, followed by addition of crystals derived from the CBD-2DMA crystals obtained in Example 1 as seed crystals.

After confirming precipitation of crystals, DMA (1 eq.) was added dropwise. The mixture was aged for crystallization at 0-5°C for 90 minutes, after which the crystals were filtered. The crystals were washed with a washing solvent [heptane: MTBE: DMA = 9:1:0.3 (volume ratio)], and dried under a nitrogen stream for 30 minutes.

The obtained crystals (solid) were confirmed to be CBD-2DMA (to contain CBD-2DMA) by the same method as above.

Heptane (4 V) and tap water (4 V) were added to the crystals (solid), and after stirring, complete dissolution of the solid was confirmed.

After liquid-liquid phase separation, the organic layer was washed with tap water (4 V), and the washed organic layer was dried with sodium sulfate (1 wt). The sodium sulfate was filtered off, the solution was washed with heptane, and then concentrated under reduced pressure.

The composition of the washed organic layer was confirmed by HPLC to be as follows: CBD 94.37%, olivetol 3.55%, abn-CBD 2.07%; Δ9-THC, Δ8-THC, and the 2:1 adduct were not detected. The quantitative value was calculated as 45.13%.

In the subsequent purification steps, the quantitative value of cannabidiol in the washed organic layer was taken as the basis for wt (mass) and V (volume).

Heptane (4.8 V) was added to the concentrated solution, and after temperature adjustment to 10°C, CBD seed crystals were added, and precipitation of crystals was confirmed. The mixture was then cooled to 0°C and crystallization aging was carried out at the same temperature. The crystals (solid) were filtered and washed with heptane cooled to the same temperature to obtain 0.7896 parts by mass of solid (crystals).

The composition of the solid (CBD crystals) was confirmed by HPLC to be as follows: CBD 94.51%, olivetol 4.80%, abn-CBD 0.69%; Δ9-THC, Δ8-THC, and the 2:1 adduct were not detected. The yield of CBD (yield assuming 100% reaction of menthadienol) was calculated as 25.6% (calculated by subtracting the mass of seed crystals).

It should be noted that an oily component was slightly exuded onto the surface of the solid. This component is considered to be olivetol.

It should be noted that, although CBD-2DMA was used as seed crystals in the above method, it has been confirmed that crystals can be obtained (crystallization or precipitation occurs) even in the absence of seed crystals.

### Example 5

### Crude Product Preparation Step

In the crude product preparation step, the mass of menthadienol used in the reaction was taken as the basis for wt (mass) and V (volume).

A reaction solution was obtained by the same method as in Synthesis Example 1.

The composition of the reaction solution was confirmed by HPLC to be as follows: CBD 62.897%, olivetol 13.764%, abn-CBD 10.695%, Δ9-THC 3.067%, Δ8-THC 0.412%, the 2:1 adduct 4.642%. The quantitative value was calculated as 64.77%.

This reaction solution was concentrated under reduced pressure to 2.49 wt, and heptane (6.6 V) was added followed by concentration under reduced pressure; this operation was repeated twice to replace the solvent with heptane.

Toluene (33 V) and silica gel (2 wt, particle size 40-50 µm, neutral, spherical) were added to the concentrated solution, and the resulting mixture was stirred at room temperature for 1 hour.

The mixture was filtered to remove the silica gel and washed with heptane (16 V), and the filtrate was concentrated under reduced pressure to 2.0 wt to obtain a crude product.

The composition of the crude product was confirmed by HPLC to be as follows: CBD 70.99%, olivetol 5.14%, abn-CBD 9.56%, Δ9-THC 4.26%, Δ8-THC 0.97%, the 2:1 adduct 6.37%. The quantitative value was calculated as 62.2%.

### Purification Step

In the purification step, the quantitative value of cannabidiol in the crude product was taken as the basis for wt (mass), V (volume), and eq. (equivalents).

Heptane (9 V) and methyl t-butyl ether (MTBE, 1 V) were added to the obtained crude product. After the resulting solution was confirmed to be a single layer, the solution was cooled to 0°C. DMA (3 eq.) was added dropwise thereto, and precipitation of crystals was confirmed.

After 1 hour of crystallization aging at 0-5°C, the crystals were filtered, washed with a washing solvent (1.5 V) [heptane: MTBE: DMA = 9:1:0.3 (volume ratio)], and then dried under a nitrogen stream for 15 minutes.

Heptane (7 V) and tap water (7 V) were added to the crystals (solid), and after stirring, complete dissolution of the solid was confirmed.

After liquid-liquid phase separation, the organic layer was washed with 10% DMA water (6 V) and tap water (12 V, twice with 6 V each), and the washed organic layer was dried with sodium sulfate (1.1 wt); the solid was filtered off and the solution was concentrated under reduced pressure.

The composition of the washed organic layer was confirmed by HPLC to be as follows CBD 93.55%, olivetol 1.59%, abn-CBD 3.97%, Δ9-THC 0.89%; Δ8-THC and the 2:1 adduct were not detected. The quantitative value was calculated as 56.27%.

### Example 6

Commercially available CBD (commercial product) was used.

According to the Certificate of Analysis (COA), the obtained CBD contains impurities derived from naturally occurring hemp (crude CBD, crude product).

Specifically, the CBD purity was 87.940%, and the main impurities (cannabinoids other than CBD) were reported to include 1.573% CBG (cannabigerol), 1.891% CBN (cannabinol), 1.055% CBC (cannabichromene), and 0.345% CBDV (cannabidivarin).

### Purification Step

In the purification step, the obtained CBD was taken as the basis for wt (mass), V (volume), and eq. (equivalents).

To the obtained CBD (crude CBD), heptane was added to adjust the weight to 7.2 wt (CBD 1 wt, heptane 6.2 wt) (equivalent to heptane 9 V), and MTBE (2 V) was added thereto, and the mixture was cooled to 10°C.

Further, DMA (3.0 eq.) was added dropwise thereto, and the mixture was cooled to -10°C. After confirming crystallization, aging (crystallization aging) was carried out for 2 hours.

The slurry after aging was filtered, and the crystals were washed with a washing solvent [a mixture of heptane (3.1 V), MTBE (0.35 V), and DMA (0.1 V)] cooled to -10°C, and then dried under a nitrogen stream over 15 minutes to obtain crystals (solid).

The filtrate obtained from the slurry was analyzed by HPLC, and impurities (CBN, etc.) were detected.

Heptane (7 V) and purified water (7 V) were added to the obtained solid (crystals), and after stirring, complete dissolution of the solid was confirmed.

After liquid-liquid phase separation, the organic layer was washed six times with purified water (7 V), and the washed organic layer was dried with sodium sulfate (1.5 wt). The sodium sulfate was filtered off, and the filtered solution was concentrated to 1.3 wt under reduced pressure.

The composition of the washed organic layer was confirmed by HPLC to be as follows: CBD 99.2%; the quantitative value was calculated as 80.6%.

In the subsequent purification steps, the quantitative value of cannabidiol in the washed organic layer was taken as the basis for wt (mass) and V (volume).

Heptane was added to the concentrated solution to adjust the weight to 2.5 wt (CBD 1 wt, heptane 1.5 wt) (equivalent to heptane 2 V), and the temperature was adjusted to 28°C.

After confirming precipitation of crystals, the slurry was cooled to -15°C over 1 hour. After crystallization aging at -15°C for 1 hour or more, the crystals (solid) were filtered and washed with heptane (1 V) cooled to the same temperature. The crystals were further dried by aeration and then dried under reduced pressure at 40°C to obtain crystals (solid).

The composition of the obtained crystals (solid) was confirmed by HPLC to be as follows: CBD 99.5%; CBG, CBN, Δ9-THC, Δ8-THC, and the like were not detected.

As is clear from the results of the Examples, according to the present invention, a solvate of cannabidiol could be obtained. Such a solvate could be obtained even via a mixture containing olivetol, other cannabinoids, or the like.

Further, by using such a solvate of cannabidiol (forming or passing through the solvate), cannabidiol could be efficiently purified (recovered).

In particular, according to the present invention, as described above, cannabidiol could be efficiently purified (recovered) even via a mixture containing olivetol, other cannabinoids, or the like.

Among them, as in Examples 2 to 3, cannabidiol could be purified (recovered) even more efficiently, by using (passing through) a mixture from which olivetol or the like had been relatively separated (removed).

In addition, as is also clear from Example 6, cannabidiol could be efficiently purified (recovered) from the mixture regardless of its origin (e.g., whether chemically synthesized or derived from natural sources, etc.).

### INDUSTRIAL APPLICABILITY

According to the present invention, a solvate of cannabidiol and the like can be provided.

## Claims

1. A solvate of cannabidiol, wherein the solvate comprises a solvent S1 comprising an aprotic polar solvent.

2. The solvate according to claim 1, wherein the solvent S1 comprises an amide-based solvent.

3. The solvate according to claim 1, wherein the solvent S1 comprises dimethylacetamide.

4. The solvate according to claim 1, which is a solvate having a molar ratio of 2 mol of dimethylacetamide relative to 1 mol of cannabidiol.

5. The solvate according to claim 1, which is in crystalline form.

6. A method for producing a solvate of cannabidiol, the method comprising:
forming a solvate from a mixture comprising cannabidiol and a solvent S1, wherein the solvent S1 of the solvate comprises an aprotic polar solvent.

7. The method according to claim 6, wherein the solvent S1 comprises an amide-based solvent.

8. The method according to claim 6, wherein the solvent S1 comprises dimethylacetamide, and the solvate is a solvate in which dimethylacetamide is solvated at a ratio of 2 mol relative to 1 mol of cannabidiol.

9. The method according to claim 6, wherein the mixture further comprises a solvent S2 different from the solvent S1.

10. The method according to claim 6, wherein the mixture further comprises an aliphatic hydrocarbon.

11. The method according to claim 6, wherein the mixture comprises dimethylacetamide and an aliphatic hydrocarbon, and precipitates a solvate in which dimethylacetamide is solvated at a ratio of 2 mol relative to 1 mol of cannabidiol.

12. The method according to claim 6, wherein the mixture further contains an additional cannabinoid.

13. The method according to claim 6, wherein the mixture contains at least one additional cannabinoid selected from the group consisting of cannabigerol, cannabinol, cannabichromene, cannabidivarin, and tetrahydrocannabinol.

14. The method according to claim 6, wherein the mixture further comprises at least one additional cannabinoid selected from the group consisting of cannabigerol, cannabinol, Δ9-tetrahydrocannabinol, and Δ8-tetrahydrocannabinol, and
the proportion of cannabigerol, cannabinol, Δ9-tetrahydrocannabinol, and Δ8-tetrahydrocannabinol relative to the total amount of cannabidiol, cannabigerol, cannabinol, Δ9-tetrahydrocannabinol, and Δ8-tetrahydrocannabinol is 0.1% or more on an HPLC area basis.

15. The method according to claim 6, wherein the mixture is derived from hemp.

16. The method according to claim 6, wherein the mixture further contains at least one selected from the group consisting of olivetol, a positional isomer of cannabidiol, an adduct of menthadienol and olivetol at a molar ratio of 2:1, Δ9-tetrahydrocannabinol, and Δ8-tetrahydrocannabinol.

17. The method according to claim 6,
wherein the mixture comprises cannabidiol and an adduct of menthadienol and olivetol at a molar ratio of 2:1,
the proportion of cannabidiol relative to the total amount of cannabidiol, olivetol, a positional isomer of cannabidiol, the adduct, Δ9-tetrahydrocannabinol, and Δ8-tetrahydrocannabinol is 60% or more on an HPLC area basis,
the mixture is free from or contains olivetol at a proportion of 0.03 or less relative to cannabidiol on an HPLC area basis,
the mixture is free from or contains the positional isomer of cannabidiol at a proportion of 0.3 or less relative to cannabidiol on an HPLC area basis, and
the mixture contains the adduct at a proportion of 0.0001 to 0.5 relative to cannabidiol on an HPLC area basis.

18. The method according to claim 6, further comprising:
subjecting a mixture comprising cannabidiol and at least one selected from the group consisting of olivetol and a positional isomer of cannabidiol to adsorption treatment to produce a purified product comprising cannabidiol, and
forming a solvate from a mixture comprising thus-obtained purified product and the solvent S1.

19. The method according to claim 6, further comprising:
reacting menthadienol with olivetol in the presence of at least one catalyst selected from the group consisting of a non-boron Lewis acid and a Bronsted acid at a temperature of 40°C or more, with a water content in the reaction system at the start of the reaction of 5 mass% or less, to produce a reaction mixture comprising cannabidiol,
subjecting thus-obtained reaction mixture to produce a purified product comprising cannabidiol, and
forming a solvate from a mixture comprising thus-obtained purified product and the solvent S1.

20. A method for producing cannabidiol comprising:
desolvating the solvate according to any one of claims 1 to 5.

21. A method for producing cannabidiol comprising:
mixing the solvate according to any one of claims 1 to 5 with water.

22. A method for extracting (liquid-liquid phase-separating) cannabidiol comprising:
mixing the solvate according to any one of claims 1 to 5, water, and a hydrophobic solvent capable of dissolving cannabidiol to extract cannabidiol into a hydrophobic solvent layer.

23. A method for producing cannabidiol, comprising:
extracting cannabidiol into a hydrophobic solvent layer by mixing the solvate according to any one of claims 1 to 5, water, and a hydrophobic solvent capable of dissolving cannabidiol; and
crystallizing the cannabidiol obtained through the extraction step.

24. Cannabidiol obtained by the method according to any one of claims 20 to 23.

25. The cannabidiol according to claim 24, wherein the proportion of cannabidiol relative to the total amount of cannabidiol, cannabigerol, cannabinol, Δ9-tetrahydrocannabinol, and Δ8-tetrahydrocannabinol is 97% or more on an HPLC area basis.

26. The cannabidiol according to claim 24, wherein the proportion of cannabidiol relative to the total amount of cannabidiol, olivetol, a positional isomer of cannabidiol, an adduct of menthadienol and olivetol at a molar ratio of 2:1, Δ9-tetrahydrocannabinol, and Δ8-tetrahydrocannabinol is 97% or more on an HPLC area basis.

27. The cannabidiol according to claim 24, which is free from or contains a solvent S1 of the solvate at a proportion of 3 mass% or less.

28. The cannabidiol according to claim 24, which contains a solvent S1 of the solvate at a proportion of 0.001 mass% or more.

29. The cannabidiol according to claim 24, which contains a solvent S1 of the solvate at a proportion of 0.001 mass% or more and an aliphatic hydrocarbon at a proportion of 0.001 mass% or more.
